(19) Europäisches Patentamt
European Patent Office
Office européen des brevets

(11) **EP 4 243 782 B1**

(12) **EUROPEAN PATENT SPECIFICATION**

(45) Date of publication and mention
of the grant of the patent:
**23.07.2025  Bulletin 2025/30**

(21) Application number: **21827249.0**

(22) Date of filing: **12.11.2021**

(51) International Patent Classification (IPC):
***A61K 9/20*** *(2006.01)*

(52) Cooperative Patent Classification (CPC):
**A61K 9/2054; A61K 9/2027; A61K 31/5025;
A61P 25/14**

(86) International application number:
**PCT/US2021/059139**

(87) International publication number:
**WO 2022/104058 (19.05.2022 Gazette 2022/20)**

(54) **TABLET FOR USE IN TREATING HUNTINGTON'S DISEASE AND METHOD OF MAKING THE SAME**

TABLETTE ZUR VERWENDUNG BEI DER BEHANDLUNG VON MORBUS HUNTINGTON UND VERFAHREN ZUR HERSTELLUNG DAVON

COMPRIMÉ DESTINÉ À ÊTRE UTILISÉ DANS LE TRAITEMENT DE LA MALADIE DE HUNTINGTON ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.11.2020  US 202063113826 P
19.09.2021  US 202163245927 P
21.09.2021  US 202163261467 P
22.09.2021  US 202163261495 P
14.10.2021  US 202163255745 P**

(43) Date of publication of application:
**20.09.2023  Bulletin 2023/38**

(73) Proprietor: **PTC Therapeutics, Inc.
Warren, NJ 07059 (US)**

(72) Inventors:
• **PINNAMANENI, Swathi
Plainfield, NJ 07080 (US)**
• **UDDIN, Akm, Nasir
Plainfield, NJ 07080 (US)**
• **DALI, Mandar, Vasant
Plainfield, NJ 07080 (US)**

(74) Representative: **Kuhn, Dieter
Novartis Pharma AG
Patent Department
Lichtstrasse 35
4056 Basel (CH)**

(56) References cited:
**WO-A1-2020/005873**

# EP 4 243 782 B1

**Description**

## CROSS-REFERENCE TO RELATED APPLICATIONS

This application claims the benefit of, and priority to pending U.S. Provisional Patent Application Serial No. 63/113,826 filed November 13, 2020, pending U.S. Provisional Patent Application Serial No. 63/245,927 filed September 19, 2021, pending U.S. Provisional Patent Application Serial No. 63/261,467 filed September 21, 2021, pending U.S. Provisional Patent Application Serial No. 63/261,495 filed September 22, 2021, and pending U.S. Provisional Patent Application Serial No. 63/255,745 filed October 14, 2021.

## FIELD OF THE INVENTION

The invention generally relates to immediate release pharmaceutical tablets of a small molecule compound for use in treating Huntington's Disease and methods of making the same.

## BACKGROUND

Huntington's Disease (HD) is a rare inherited neurodegenerative disorder caused by a mutation in the *huntingtin* (*HTT*) gene. The disorder results in behavioral, cognitive, and motor impairments. These symptoms progressively reduce an individual's quality of life, and ultimately lead to death within 15 to 25 years of overt clinical motoric onset. Each child of a parent with a mutation in the *huntingtin* gene has a 50% chance of inheriting the mutation. It is estimated that around one person in 10,000 carries the mutated huntingtin gene. Current HD therapies manage the severity of the symptoms, but there are currently no therapeutics approved that slow the progression of the disease.

HD is caused by CAG repeat expansions in *HTT* and is characterized by motor, cognitive, psychiatric and functional capacity decline. The CAG trinucleotide repeat expansion results in a mutant huntingtin protein (mHTT), which is associated with neural dysfunction and ultimately death.

The number of CAG repeats in the *HTT* gene ranges from 6 to 35 in healthy individuals. Disease penetrance is seen to be reduced for individuals carrying 36 to 39 CAG repeats, however those with 40 or more CAG repeats are almost certain to develop the disease. As described in European Journal of Neurology, 2017, 24-34, clinical diagnosis of HD is based on: confirmed family history or positive genetic test (i.e. confirmation of CAG repeat expansion ≥36); and onset of motor disturbance as defined by the Unified Huntington's Disease Rating Scale (UHDRS), with a total motor score (TMS) diagnostic confidence score (DCS), which ranges from 0 (no motor abnormalities suggestive of HD) to 4 (motor abnormalities ≥99% likely to be due to HD), wherein a score of 4 defines "motor onset" or "manifest" HD.

Typically, age of onset (i.e. once the DCS reaches 4) ranges between 30 to 50 years and average duration of survival after clinical diagnosis is 15 to 20 years. Currently, after onset, loss of "function" (i.e. assessment of functional capacities), rather than motor signs, determines disease stage (see, e.g. Neurology, 1979, 29, 1-3; or Neurology, 1981, 31, 1333-1335). The Total Functional Capacity (TFC) scale (see, e.g. Movement Disorders, 1996, 11, 136-142) is a component of the UHDRS, where the level of independence of a person with HD ranges from 0 (fully dependent for all care) to 13 (fully independent). This scale assesses functional status of a HD patient in terms of ability to work, handle household finances, manage domestic chores, perform activities of daily living, and level of care needed. Based on the UHDRS total functional capacity (TFC), HD is divided into stages 1 to 5 of disease progression. The categorization of HD, based on TFC score (also referred to as Shoulson and Fahn stages), are also described as early stage of HD (corresponding to stages 1 or 2, based on TFC score), moderate stage or mid stage HD (corresponding to stage 3, based on TFC score) and advanced stage or late stage HD (corresponding to stage 4 or 5, based on TFC score).

An international application published as WO2020/005873 identified a genus of compounds that can be used in the treatment of HD, methods of making the same and pharmaceutical formulations of the same. It also provided data showing that the compounds inhibited endogenous Huntington protein (HTT), in an $IC_{50}$ assay. One of the compounds disclosed in that application as a particularly potent inhibitor of HTT, 2-[3-(2,2,6,6-tetramethylpiperidin-4-yl)-3H-[1,2,3]triazolo[4,5-c]pyridazin-6-yl]-5-(2H-1,2,3-triazol-2-yl)phenol has since been found to be effective in reducing human HTT production *in vivo* in transgenic mouse models of Huntington's disease (results not yet published). At present, only symptomatic treatments are available. Thus, to date, there is no small molecule therapy available to slow the progression of HD. Accordingly, there is a need for small molecule disease-modifying therapies for HD (i.e. therapeutic options that can slow disease progression).

## SUMMARY OF THE INVENTION

In one aspect the invention relates to a tablet comprising, as an active ingredient, 2-[3-(2,2,6,6-tetramethylpiperidin-4-yl)-3H-[1,2,3]triazolo[4,5-c]pyridazin-6-yl]-5-(2H-1,2,3-triazol-2-yl)phenol (hereinafter Compound 1), or a pharmaceutically acceptable salt thereof, wherein Compound 1 is present in an amount of from about 1% to about 30% by weight of the total weight of the tablet, an intragranular excipient, and an extragranular excipient,

wherein the intragranular excipient comprises microcrystalline cellulose and a diluent, wherein the ratio of microcrystalline cellulose to diluent is about 1:1 to about 1:4 and microcrystalline cellulose is present in an amount of about 15% to about 25% by weight of the total weight of the tablet, a disintegrant in an amount of about 1% to 3% of the total weight of the tablet, and povidone in an amount of about 1% to about 5% by weight of the total weight of the tablet, and

wherein the extragranular excipient comprises an additional amount of the diluent and an additional amount of the disintegrant.

In one aspect Compound 1 is present in an amount of about 5% to about 25% of the total weight of the tablet. In another aspect Compound 1 is present at about 10% of the total weight of the tablet.

In one aspect, the amount of Compound 1 in the tablet is in a range from 1 mg to 200 mg.

In another aspect, the amount of Compound 1 in the tablet is in a range from 1 mg to 100 mg.

In another aspect, the amount of Compound 1 in the tablet is selected from 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, and 200 mg.

In another aspect, the amount of Compound 1 in the tablet is selected from 1 mg, 5 mg, 10 mg, 15 mg, 20 mg, 25 mg, 30 mg, 35 mg, 50 mg, 60 mg, 65 mg, 70 mg, 75 mg, 80 mg, 85 mg, 90 mg, 95 mg, 100 mg, 110 mg, 120 mg, 135 mg, and 140 mg.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, 75 mg, 80 mg, 85 mg, 90 mg, 95 mg, 100 mg, 110 mg, 120 mg, 135 mg, and 140 mg.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, and 100 mg.

In another aspect, the amount of Compound 1 in the tablet is selected from 1 mg, 5 mg, 10 mg, 20 mg, 30 mg, and 50 mg.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, and 100 mg.

In another aspect, the amount of Compound 1 in the tablet is selected from 1 mg, 5 mg, 10 mg, 20 mg, 30 mg, and 50 mg.

In another aspect, the amount of Compound 1 in the tablet is selected from 1 mg, 5 mg or 50 mg.

In another aspect, the amount of Compound 1 in the tablet is selected from 5 mg or 50 mg.

In another aspect, the amount of Compound 1 in the tablet is selected from 5 mg, 10 mg, 20 mg, and 30 mg.

In another aspect, the amount of Compound 1 in the tablet is selected from 5 mg, 10 mg, and 20 mg.

In one aspect, the diluent is lactose monohydrate.

In one aspect, the disintegrant is croscarmellose sodium.

In one aspect, the ratio of microcrystalline cellulose to diluent in the intragranular excipient is about 1 to 2.

In one aspect, at least one of the extragranular excipient and intragranular excipient further comprises a surfactant. In one such aspect, the surfactant is a poloxamer.

In one aspect, the disintegrant is croscarmellose sodium.

In one aspect, the extragranular excipient further comprises a lubricant. In one such aspect, the lubricant is magnesium stearate.

In one aspect, the extragranular excipient further comprises a glidant. In one such aspect, the glidant is colloidal silicon dioxide.

In one aspect, the total weight of the extragranular excipient is from about 15% to about 30% of the total weight of the tablet.

In one aspect, the intragranular excipients have been wet granulated.

In another aspect, the tablet comprises Compound 1 in an amount of about 10% by weight of the tablet, the intragranular excipient comprises microcrystalline cellulose and lactose monohydrate in a ratio of about 1:2 and the microcrystalline cellulose is present in an amount of about 20% by weight of the tablet, the disintegrant in an amount of 1% to about 3% by weight of the tablet, and the povidone in an amount of about 2% by weight of the tablet, and the extragranular excipient comprises an additional amount of lactose monohydrate in an amount of about 10% to about 25% of the weight of the tablet, an additional amount of the disintegrant in an amount of about 1% to about 5% by weight of the tablet, and poloxamer in an amount of about 0.5% to about 2% by weight of the tablet.

In another aspect the tablet further comprises colloidal silicon dioxide in an amount of about 0.25% to about 2% by weight of the tablet.

In another aspect, the tablet further comprises magnesium stearate in an amount of about 0.5% to about 2% by weight of the tablet.

The invention also relates to a method of making the tablet comprising wet granulating the intragranular excipients, drying the resulting intragranular blend, mixing the extragranular excipient with the intragranular excipient, and compressing the resulting mixture to form a tablet.

In another aspect, the method further comprises as step of coating the tablet with a film.

The invention also relates to a tablet according to any of the claims 1-13 for use in a method of treating or ameliorating Huntington's Disease in a subject in need thereof, comprising administering to the subject a tablet having a therapeutically effective amount of Compound 1, or a pharmaceutically acceptable salt thereof.

In another aspect, the invention also relates to the use of Compound 1, or a pharmaceutically acceptable salt thereof, in a treatment slowing progression of Huntington's disease; wherein, the effect of treatment with Compound 1, or a pharmaceutically acceptable salt thereof, slows progression of Huntington's disease by producing an in-frame stop codon between exons 49 and 50 in the HTT mRNA; wherein, the effect of treatment with Compound 1, or a pharmaceutically acceptable salt thereof, slowing progression of Huntington's disease is a disease-modifying therapy; wherein, the effect of treatment with Compound 1, or a pharmaceutically acceptable salt thereof, slows the decline of motor function associated with Huntington's disease; wherein, the effect of treatment with Compound 1, or a pharmaceutically acceptable salt thereof, slows cognitive decline associated with Huntington's disease; wherein, the effect of treatment with Compound 1, or a pharmaceutically acceptable salt thereof, slows psychiatric decline associated with Huntington's disease; wherein, the effect of treatment with Compound 1, or a pharmaceutically acceptable salt thereof, slows the decline of functional capacity associated with Huntington's disease; wherein, the effect of treatment with Compound 1, or a pharmaceutically acceptable salt thereof, slows the progression of Huntington's disease pathophysiology.

## BRIEF DESCRIPTION OF THE DRAWINGS

Figure 1 is a plot of individual plasma concentrations of Compound 1 over time after oral administration of a Compound 1 suspension formulation (Batch 21) in 0.5% hydroxypropyl methyl cellulose (HPMC) in water at 30 mg in Male Cynomolgus Monkeys (Leg 1)

Figure 2 is a plot of mean plasma concentrations of Compound 1 over time after oral administration of a Compound 1 suspension (Batch 21) in 0.5% HPMC in water at 30 mg in Male Cynomolgus Monkeys (Leg 1).

Figure 3 is a plot of individual plasma concentrations of Compound 1 over time after oral administration of Tablet Formulation A (wet granulation Batch 15) at 30 mg in Male Cynomolgus Monkeys (Leg 2).

Figure 4 is a plot of mean plasma concentrations of Compound 1 over time after oral administration of Tablet Formulation A (wet granulation Batch 15) at 30 mg in Male Cynomolgus Monkeys (Leg 2).

Figure 5 is a plot of individual plasma concentrations of Compound 1 over time after oral administration of Tablet Formulation B (dry granulation Batch 20) at 30 mg in Male Cynomolgus Monkeys (Leg 3).

Figure 6 is a plot of the mean plasma concentrations of Compound 1 over time after oral administration of Tablet Formulation B (dry granulation Batch 20) at 30 mg in Male Cynomolgus Monkeys (Leg 3).

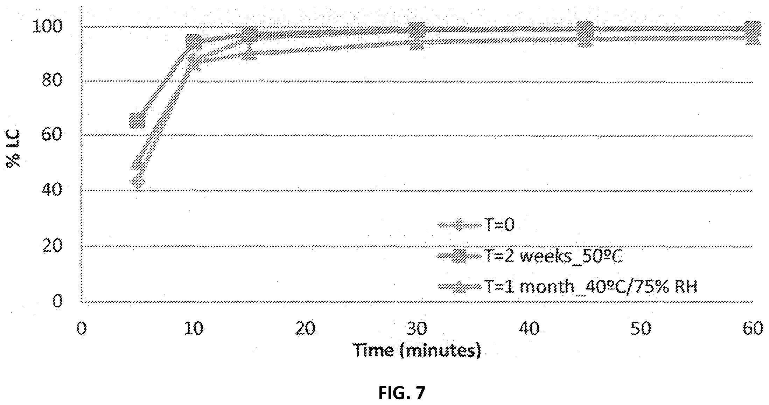

Figure 7 is dissolution profiles (% dissolved Compound 1 over time) of 5 mg tablets produced from Batch 23 before and after storage at 2 weeks at 50°C or 1 month at 40°C/75% relative humidity.

Figure 8 is dissolution profiles (% dissolved Compound 1 over time) of 50 mg tablets produced from Batch 23 before and after storage at 2 weeks at 50°C or 1 month at 40°C/75% relative humidity.

Figure 9 shows a dose-dependent reduction in HTT mRNA in whole blood taken from healthy volunteers participating in a Single Ascending Dose (SAD) and Multiple Ascending Dose study of a Phase I clinical trial.

Figure 9A shows a lowering of HTT mRNA in whole blood taken from healthy volunteers in the SAD cohort where splicing was evaluated 24 hours after they were administered a one day, single dose of either placebo, 5 mg, 15 mg, 45 mg, 90 mg, or 135 mg of Compound 1.

Figure 9B shows the lowering of HTT mRNA in whole blood taken from healthy volunteers in the MAD cohort dosed daily with either placebo, 15 mg or 30 mg of Compound 1 for 14 days. HTT splicing was then evaluated by RT-PCR 6 hours after administration of Compound 1 on day 14.

Figure 10 shows how decay rates can be modeled to predict drug-dependent decrease in mRNA and protein Concentration over time.

Figure 11 shows graphs that model the rate of HTT mRNA (Figure 11A) and HTT protein (Figure 11B) decay based on their half-lives and then predicted the time to reach steady state after Compound 1 treatment at 30 mg daily dose. For HTT mRNA, the half-life is estimated to be about 24 hours. HTT mRNA in Figure 11A reaches steady state after approximately 5 days. For HTT protein, the half-life is estimated to be 5-7 days and consequently HTT protein steady state levels should take about 6 weeks from the beginning of treatment.

Figure 12 compares the trajectory of HTT mRNA (Figure 12A) and protein (Figure 12B) lowering seen in Multiple Ascending Dose Study with those values predicted from the half-life of HTT mRNA and protein as shown in Figure 11.

Figure 13 shows that Compound 1 crosses the Blood Brain Barrier in non-human primates (Figure 13A) and in humans (Figure 13B).

Figure 14 is a plot of % of baseline of *HTT* RNA measured over time in whole blood of human subjects administered a placebo or a single dose of 90 mg of Compound 1, as described in the Single Ascending Dose (SAD) study in Part 1 of Example 10. The results show that the *HTT* splicing effect of Compound 1 is reversible and persists for 72 hours post cessation of treatment.

Figure 15 is a plot of % baseline of *HTT* RNA measured over time in the whole blood of human subject administered a placebo or 15 or 30 mg of Compound 1, as described in the Multiple Ascending Dose (MAD) study described in Part 2 of Example 10. *HTT* splicing was monitored after the final dose at day 14, calculated as % *HTT* remaining from baseline (pre-dose day 0).

Figure 16 is a bar graph showing the huntingtin mRNA and protein levels in whole blood from MAD cohort 2.3 (30 mg administered for 21 days with 100 mg loading dose (LD) for 2 days), as described in Example 10, as a percent of baseline, after administration of vehicle or compound 1 to a human, 24 hours after the last dose. The results show *HTT* mRNA reduction reached steady state. Longer dosing was required for HTT protein levels to reach maximal steady state reduction.

## DETAILED DESCRIPTION OF THE INVENTION

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, the materials, methods, and examples are illustrative only and not intended to be limiting. Other features of the disclosure are apparent from the following detailed description and the claims.

Titles or subtitles may be used in the specification for the sole convenience of the reader but are not intended to influence the scope of the present disclosure or to limit any aspect of the disclosure to any subsection, subtitle, or paragraph.

### 1. Definitions

As used herein, the singular forms "a," "an," and "the," are intended to include the plural forms as well, unless the context clearly indicates otherwise.

As used herein and in the claims, the phrase "at least one," in reference to a list of one or more elements, should be understood to mean at least one element selected from any one or more of the elements in the list of elements, but not necessarily including at least one of each and every element specifically listed within the list of elements, and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one aspect, to at least one, optionally including more than one, A, with no B present (and optionally including elements other than B); in another aspect, to at least one, optionally including more than one, B, with no A present (and optionally including elements other than A); in yet another aspect, to at least one, optionally including more than one, A, and at least one, optionally including more than one, B (and optionally including other elements); etc.

When the term "about" is used in conjunction with a numerical range, it modifies that range by extending the boundaries above and below those numerical values. In general, the term "about" is used herein to modify a numerical value above and below the stated value by a variance of 20%, 10%, 5%, or 1%. In certain aspects, the term "about" is used to modify a numerical value above and below the stated value by a variance of 10%. In certain aspects, the term "about" is used to modify a numerical value above and below the stated value by a variance of 5%. In certain aspects, the term "about" is used to modify a numerical value above and below the stated value by a variance of 1%.

The terms "subject" or "patient" are used interchangeably to refer to an individual human suffering from a disease described herein (e.g. Huntington's Syndrome) that can be treated by administration of a composition described herein.

When a range of values is listed herein, it is intended to encompass each value and subrange within that range. For example, "1-5 ng" or a range of "1 ng to 5 ng" is intended to encompass 1 ng, 2 ng, 3 ng, 4 ng, 5 ng, 1-2 ng, 1-3 ng, 1-4 ng, 1-5 ng, 2-3 ng, 2-4 ng, 2-5 ng, 3-4 ng, 3-5 ng, and 4-5 ng.

It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

The terms "treat," "treatment," "treating" refer to therapeutic treatments, wherein the object is to reverse, alleviate, ameliorate, inhibit, slow down or stop the progression or severity of a disorder. The term "treating" includes, in the alternative, the term "ameliorating," which refers to reducing or alleviating at least one adverse effect or symptom of a condition, disease or disorder. Treatment is generally "effective" if one or more symptoms or clinical markers are reduced. Alternatively, treatment is "effective" if the progression of a disorder is slowed, reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

The term "excipient" as used herein means any substance, not itself a therapeutic agent, used as a carrier or vehicle for delivery of a therapeutic agent to a subject or added to a pharmaceutical composition to improve its handling or storage properties or to permit or facilitate formation of the dose unit of the composition into a discrete article, such as a capsule or tablet suitable for oral administration. Excipients include, by way of illustration and not limitation, diluents, disintegrants, binding agents, adhesives, surfactants, lubricants, glidants, surface modifying agents, substances added to mask or counteract a disagreeable taste or odor, flavors, dyes, fragrances, and substances added to improve the appearance of the composition.

The terms "HD" or "Huntington's disease", as used herein, refer to the neurodegenerative disorder, characterized by motor, cognitive, psychiatric and functional capacity decline, and caused by CAG repeat expansions in the huntingtin gene.

The term "intragranular" as used herein refers to ingredients that are incorporated into a formulation prior to granulation, i.e. ingredients that are located internally in or part of the granule structure.

The term "extragranular" as used herein, refers to ingredients that are incorporated into a formulation after granulation, i.e. ingredients that are located externally to the granule structure.

The terms "administer" or "administration" as used herein, refer to the act of physically delivering a substance as it exists outside the body into a subject.

The terms "manifest HD" or "manifest Huntington's disease", as used herein, refer to having diagnosis of HD as clinically established {e.g. on the basis of: confirmed family history or positive genetic test (confirmation of CAG repeat expansion ≥36); and onset of motor disturbances [diagnostic confidence score (DCS) of 4, as defined by the Unified Huntington Rating Scale (UHDRS) total motor score (TMS)]. In one aspect, the term "manifest HD" or "manifest Huntington's disease", as used herein, refers to a patient having diagnosis of HD as clinically established [e.g. on the basis of confirmed family history or positive genetic test (confirmation of CAG repeat expansion ≥36)]; and onset of motor disturbances [e.g. on the basis of diagnostic confidence score (DCS) of 4, as defined by the Unified Huntington Rating Scale (UHDRS) total motor score (TMS)].

The terms "pre-manifest HD" or "pre-manifest Huntington's disease", as used herein, refer to having genetic diagnosis of HD [e.g. on the basis of: positive genetic test (confirmation of CAG repeat expansion ≥40) without onset of motor disturbances as clinically stablished, for example, as assessed according to standard scales, such as, clinical scales [e.g. on the basis of a diagnostic confidence score (DCS) of <4, as defined by the Unified Huntington Rating Scale (UHDRS) total motor score (TMS)]. In one aspect, term "pre-manifest HD" or "pre-manifest Huntington's disease", as used herein, refers to a patient having genetic diagnosis of HD [e.g. on the basis of: positive genetic test (confirmation of CAG repeat expansion ≥40)] without onset of motor disturbances as clinically stablished, for example, as assessed according to standard scales, such as, clinical scales [e.g. on the basis of a diagnostic confidence score (DCS) of <4, as defined by the Unified Huntington Rating Scale (UHDRS) total motor score (TMS)].

The terms "slowing progression of HD", "slowing progression of Huntington's disease", "to slow the progression of HD" or "to slow the progression of Huntington's disease", as used herein, refer to, one or more treatment effects selected from reducing the rate of Huntington's disease progression (e.g. reducing the rate of progression between stages of Huntington's disease); delaying the onset of Huntington's disease; delaying the onset of symptoms associated with Huntington's disease; reducing the rate of progression (e.g. reducing the annual rate of decline) of symptoms (e.g. one or more symptoms) associated with Huntington's disease; or reducing the rate of progression of Huntington's disease pathophysiology (e.g. treatment effects compared to placebo or compared to natural history control group; e.g. according to standard scales, such as clinical scales, herein above or below, or according to neuroimaging measures).

The term "rate of progression", as used herein, refers, for example, to the annual rate of change (e.g. decline) or the rate of change (e.g. decline) per year, for example as assessed according to standard scales, such as clinical scales, or according to neuroimaging measures.

The term "reducing", as used herein, refers to e.g. 5%, 10%, 20%, 30%, 40%, 50%, 60% or 70% reduction, for example, per year of treatment.

The term "delaying", as used herein, refers to delay for at least e.g. 0.5, 1, 2, 3, 4, 5, 6, 7, 8, 9, 10, 11, 12, 13, 14 or 15 years.

The terms "slowing progression of HD", "slowing progression of Huntington's disease", "to slow the progression of HD" or "to slow the progression of Huntington's disease", as used herein, refer to delaying the onset of Huntington's disease, e.g. increasing time for the onset of Huntington's disease as defined herein. In another aspect, the terms refer to reducing the rate of progression between stages of Huntington's disease, for example, reducing the rate of progression from an initial stage of HD into a more advanced stage of HD, as assessed, for example, compared to placebo, according to standard scales, such as clinical scales [e.g. according to the UHDRS total functional capacity (TFC) scale, for example, in Neurology, 1979, 29, 1-3]. In another aspect, it refers to reducing the rate of progression from stage 1 of HD into stage 2 of HD (e.g. compared to placebo). In another aspect, the terms refer to reducing the rate of progression from stage 2 of HD into stage 3 of HD (e.g. compared to placebo). In another aspect, the terms refer to reducing the rate of progression from stage 3 of HD into stage 4 of HD (e.g. compared to placebo). In another aspect, the terms refer to reducing the rate of progression from stage 4 of HD into stage 5 of HD (e.g. compared to placebo). In another aspect, the terms refer to reducing the rate of progression from early HD into middle stage HD (e.g. compared to placebo). In another aspect, the terms refer to reducing the rate of progression from middle stage HD into advanced HD (e.g. compared to placebo).

The term "reducing the rate of progression", as used herein, refers, for example, to increasing time for progression of stage of HD (e.g. compared to placebo).

The terms "slowing progression of HD", "slowing progression of Huntington's disease", "to slow the progression of HD" or "to slow the progression of Huntington's disease", as used herein, refer to delaying the onset of Huntington's disease (e.g. increasing time for the onset of Huntington's disease as defined herein) by at least 25% (e.g. by 25% or more, such as from 25% to 50%).

The term "onset of Huntington's disease", as used herein, refers to clinical diagnosis of HD as generally established [e.g. onset of motor disturbances based on diagnostic confidence score (DCS) of 4, as defined by the Unified Huntington Rating Scale (UHDRS) total motor score (TMS)].

The terms "slowing progression of HD", "slowing progression of Huntington's disease", "to slow the progression of HD" or "to slow the progression of Huntington's disease", as used herein, refer to delaying the onset of symptoms associated with Huntington's disease, e.g. increasing time for the onset of one or more symptom associated with Huntington's disease selected from decline of motor function associated with Huntington's disease, cognitive decline associated with Huntington's disease, psychiatric decline associated with Huntington's disease and decline of functional capacity associated with Huntington's disease, as defined herein. In another aspect, the terms refer to reducing the rate of progression of one or more symptom associated with Huntington's disease selected from decline of motor function associated with Huntington's disease, cognitive decline associated with Huntington's disease, psychiatric decline associated with Huntington's disease and decline of functional capacity associated with Huntington's disease, as defined herein. The term "reducing the rate of, as used herein, refers, for example, to increasing time for onset or increasing time for a rise of severity (e.g. compared to placebo). In another aspect, the terms "slowing progression of HD", "slowing progression of Huntington's disease", "to slow the progression of HD" or "to slow the progression of Huntington's disease", as used herein, refer to reducing the rate of progression of pre-manifest HD into manifest HD [i.e. delaying the onset of manifest HD; e.g. compared to placebo; e.g. as assessed by a diagnostic confidence score (DCS) of 4, as defined by the Unified Huntington Rating Scale (UHDRS) total motor score (TMS)].

The terms "slowing progression of HD", "slowing progression of Huntington's disease", "to slow the progression of HD" or "to slow the progression of Huntington's disease", as used herein, refer to slowing the progression of Huntington's disease pathaphysiology.

The term "slowing the progression of Huntington's disease pathophysiology", as used herein, refers to reducing the rate of progression of Huntington's disease pathophysiology, for example, as assessed by magnetic resonance imaging (MRI) [e.g. by neuroimaging measures, such as in Lancet Neural. 2013, 12 (7), 637-649]. For example, it refers to reducing the rate (e.g. reducing the annual rate, for example, versus placebo) of brain (e.g. whole brain, caudate, striatum or cortex) volume loss (e.g. % from baseline volume) associated with Huntington's disease (e.g. as assessed by MRI).

The term "motor function", as used herein, refers to motor features of HD comprising, for example, one or more selected from the group consisting of ocular motor function, dysarthria, chorea, postural stability and gait.

The term "decline of motor function", as used herein, refers to decreased motor function (e.g. from normal motor function or from previous clinic visit). Decline of motor function may be assessed, for example, according to standard scales, such as clinical scales (e.g. UHDRS motor assessment scale, as measured by the UHDRS Total Motors Score; e.g. in Movement Disorders, 1996, 11, 136-142).

The terms "slowing the decline of motor function" or "to slow the decline of motor function", as used herein, refer to reducing the rate of decline of motor function (e.g. compared to placebo; e.g. reduction in the annual rate of decline of motor function, for example, versus placebo; e.g. as assessed by the UHDRS Total Motors Score).

The term "reducing the rate", as used herein, refers to increasing time for onset or increasing time for a rise of severity (e.g. compared to placebo; e.g. reduction in the annual rate of decline, for example, versus placebo).

The term "cognitive decline", as used herein, refers to decreased cognitive abilities (e.g. from normal cognition function or from previous clinic visit). In one aspect, the term refers to, for example, decline of one or more cognition functions selected from the group consisting of attention, processing speed, visuospatial processing, timing, emotion processing, memory, verbal fluency, psychomotor function, and executive function. Cognitive decline may be assessed, for example, according to standard scales, such as clinical scales [e.g. as assessed by the Symbol Digit Modalities Test, the Stroop Word Reading Test, the Montreal Cognitive Assessment or the HD Cognitive Assessment Battery (comprising the Symbol Digit Modalities Test, Trail Making Test B, One Touch Stockings, Paced Tapping, Emotion Recognition Test, Hopkins Verbal Learning Test); e.g. in Movement Disorders, 2014, 29 (10), 1281-1288].

The terms "slowing cognitive decline" or "to slow cognitive decline", as used herein, refer to reducing the rate of cognitive decline (e.g. compared to placebo; e.g. reduction in the annual rate of cognitive decline versus placebo; e.g. as assessed by the Symbol Digit Modalities Test, by the Stroop Word Reading Test, by the Montreal Cognitive Assessment or by the HD Cognitive Assessment Battery). The term "reducing the rate", as used herein, refers to increasing time for onset or increasing time for a rise of severity (e.g. compared to placebo; e.g. reduction in the annual rate of decline, for example, versus placebo).

The term "psychiatric decline", as used herein, refers to decreased psychiatric function (e.g. from normal psychiatric function or from previous clinic visit). In one aspect, the term refers to, for example, one or more psychiatric functions selected from the group consisting of apathy, anxiety, depression obsessive compulsive behavior, suicidal thoughts, irritability and agitation. Psychiatric decline may be assessed, for example, according to standard scales, such as clinical scales (e.g. as assessed by the Apathy Evaluation Scale or by the Hospital Anxiety and Depression Scale; e.g. in Movement Disorders, 2016, 31 (10), 1466-1478, Movement Disorders, 2015, 30 (14), 1954-1960).

The terms "slowing psychiatric decline" or "to slow psychiatric decline", as used herein, refer to reducing the rate of psychiatric decline (e.g. compared to placebo; e.g. reduction in the annual rate of psychiatric decline versus placebo; e.g. as assessed by the Apathy Evaluation Scale or by the Hospital Anxiety and Depression Scale). The term "reducing the rate", as used herein, refers to increasing time for onset or increasing time for a rise of severity (e.g. compared to placebo; e.g. reduction in the annual rate of decline, for example, versus placebo).

The term "functional capacity", as used herein, refers, for example, to the ability to work, handle financial affairs, manage domestic chores, perform activities of daily living, and level of care needed. Functional capacity comprises, for example, one or more selected from the group consisting of capacity to work, capacity to handle financial affairs, capacity to manage domestic chores, capacity to perform activities of daily living, and level of care needed.

The term "decline of functional capacity", as used herein, refers to decreased functional capacity (e.g. from normal functional capacity or from previous clinic visit). Decline of functional capacity may be assessed, for example, according to standard scales, such as clinical scales (e.g. UHDRS functional assessment scale and independence scale, and UHDRS Total Functional Capacity Scale e.g. in Movement Disorders, 1996, 11, 136-142).

The terms "slowing the decline of functional capacity" or "to slow the decline of functional capacity", as used herein, refer to reducing the rate of decline of functional capacity (e.g. compared to placebo; e.g. reduction in the annual rate of decline of functional capacity versus placebo; e.g. as assessed by the UHDRS functional assessment scale and independence scale or by the UHDRS Total Functional Capacity Scale). The term "reducing the rate", as used herein, refers to increasing time for onset or increasing time for a rise of severity (e.g. compared to placebo; e.g. reduction in the annual rate of decline, for example, versus placebo).

The term "decline", as used herein, refers, for example, to worsening over time (e.g. annually or per year) of a condition or of a particular feature of a condition, for example as assessed according to standard scales, such as clinical scales.

The terms "Unified Huntington Disease Rating Scale" or "UHDRS" as used herein, refer to the clinical rating scale developed by the Huntington Study Group (e.g. in Movement Disorders, 1996, 11, 136-142), which assesses domains of clinical performance and capacity in HD. The UHDRS comprises rating scales for motor function, cognitive function and functional capacity. It yields scores assessing primary features of HD (e.g. motor and cognitive) and overall functional impact of these features.

The term "cHDRS" refers to the composite Unified Huntington Disease Rating Scale, which provides composite measure of motor, cognitive and global functioning (e.g. in Neurology, 2017, 89, 2495-2502).

The terms "HD stage 1", "HD stage I", "Huntington's disease stage 1", "Huntington's disease stage I", "stage 1 of Huntington's disease" or "stage I of Huntington's disease", as used herein, refer to a disease stage of HD as clinically stablished [e.g. as assessed according to standard scales, for example, clinical scales, such as on the basis of the UHDRS total functional capacity (TFC) scale, wherein the TFC score is from 11 to 13]. At HD stage 1, typically, the patient has been clinically diagnosed with HD, is fully functional at home and at work and maintains independence as regards functional capacities; typically 0 to 8 years from onset of Huntington's disease.

The terms "HD stage 2", "HD stage II", "Huntington's disease stage 2", "Huntington's disease stage II", "stage 2 of Huntington's disease" or "stage II of Huntington's disease", as used herein, refer to a disease stage of HD as clinically stablished [e.g. as assessed according to standard scales, for example, clinical scales, such as on the basis of the UHDRS total functional capacity (TFC) scale, wherein the TFC score is from 7 to 10]. At HD stage 2, typically, the patient is still functional at work, however at lower capacity, is mostly able to carry out daily activities, despite some difficulties, and usually requires only slight assistance; typically 3 to 13 years from onset of Huntington's disease.

The terms "HD stage 3", "HD stage III", "Huntington's disease stage 3", "Huntington's disease stage III", "stage 3 of Huntington's disease" or "stage III of Huntington's disease", as used herein, refer to a disease stage of HD as clinically stablished [e.g. as assessed according to standard scales, for example, clinical scales, such as on the basis of the UHDRS total functional capacity (TFC) scale, wherein the TFC score is from 4 to 6]. At HD stage 3, typically, the patient can no longer conduct work or manage household chores, requires substantial help for daily financial affairs, domestic responsibilities, and activities of daily living; typically 5 to 16 years from onset of Huntington's disease.

The terms "HD stage 4", "HD stage IV", "Huntington's disease stage 4", "Huntington's disease stage IV", "stage 4 of Huntington's disease" or "stage IV of Huntington's disease", as used herein, refer to a disease stage of HD as clinically stablished [e.g. as assessed according to standard scales, for example, clinical scales, such as on the basis of the UHDRS total functional capacity (TFC) scale, wherein the TFC score is from 1 to 3]. At HD stage 4, typically, the patient is not independent, but still can reside at home with help from either family or professionals, however, requiring substantial assistance in financial affairs, domestic chores, and most activities of daily living; typically 9 to 21 years from onset of Huntington's disease.

The terms "HD stage 5", "HD stage V", "Huntington's disease stage 5", "Huntington's disease stage V", "stage 5 of Huntington's disease" or "stage V of Huntington's disease", as used herein, refer to a disease stage of HD as clinically stablished [e.g. as assessed according to standard scales, for example, clinical scales, such as on the basis of the UHDRS total functional capacity (TFC) scale, wherein the TFC score is 0]. At HD stage 5, typically, the patient needs total support in daily activities from professional nursing care; typically 11 to 26 years from onset of Huntington's disease.

The terms "early HD", "early Huntington's disease", "early stage of HD" or "early stage of Huntington's disease", as used herein, refer to a disease stage of HD, wherein the patient is largely functional and may continue to work and live independently, despite suffering from, for example, one or more selected from the group consisting of minor involuntary movements, subtle loss of coordination and difficulty thinking through complex problems. In another aspect, the terms "early HD", "early Huntington's disease", "early stage of HD" or "early stage of Huntington's disease", refer to "HD stage 2", as defined herein.

The terms "moderate HD", "moderate Huntington's disease", "moderate stage of HD", "moderate stage of Huntington's disease", "middle stage HD", "middle stage Huntington's disease", "middle stage of HD" or "middle stage of Huntington's disease", as used herein, refer to a disease stage of HD, wherein the patient may no be able to work, manage own finances or perform own household chores, but will be able to eat, dress, and attend to personal hygiene with assistance. Typically, at this stage, for example, chorea may be prominent, as well as problems with swallowing, balance, falls, weight loss, and problem solving. In another aspect, the terms "moderate HD", "moderate Huntington's disease", "moderate stage of HD", "moderate stage of Huntington's disease", "middle stage HD", "middle stage Huntington's disease", "middle stage of HD" or "middle stage of Huntington's disease" refer to "HD stage 3", as defined herein.

The terms "advanced HD", "advanced Huntington's disease", "advanced stage of HD", "advanced stage of Huntington's disease", "late HD" or "late Huntington's disease", "late stage of HD" or "late stage of Huntington's disease", as used herein, refer to a disease stage of HD, wherein the patient requires assistance in all activities of daily living. Typically, at this stage, for example, chorea may be severe, but more often it is replaced by rigidity, dystonia, and bradykinesia. In another aspect, the terms "advanced HD", "advanced Huntington's disease", "advanced stage of HD", "advanced stage of Huntington's disease", "late HD" or "late Huntington's disease", "late stage of HD" or "late stage of Huntington's disease" refers to "HD stage 4" or "HD stage 5", as defined herein.

The terms "juvenile HD" or "juvenile Huntington's disease", as used herein, refer to diagnosis of HD as clinically stablished {e.g. on the basis of: confirmed family history or positive genetic test (i.e. confirmation of CAG repeat expansion 2:36); and onset of symptoms by age< 21 years}.

The terms "juvenile HD" or "juvenile Huntington's disease", as used herein, refer to a patient affected by HD {e.g. on the basis of: confirmed family history or positive genetic test (i.e. confirmation of CAG repeat expansion 2:36)} and who has onset of symptoms by age< 21 years.

The terms "pediatric HD" or "pediatric Huntington's disease", as used herein, refer to a patient affected by HD {e.g. on the basis of: confirmed family history or positive genetic test (i.e. confirmation of CAG repeat expansion 2:36) and clinical diagnosis} and who is aged <18 years.

The terms "HD patient", "Huntington's disease patient", "patient with Huntington's disease" or "patient with HD" refer to a patient with HD, as defined herein.

The terms "treat" "treating" "treatment" or "therapy", as used herein, refer to obtaining beneficial or desired results, for example, clinical results. Beneficial or desired results can include, but are not limited to, stabilizing or improving progression of stage of HD (e.g. compared to placebo). One aspect of the treatment is, for example, that said treatment should have a minimal adverse effect on the patient, e.g. the agent used should have a high level of safety, for example without producing adverse side effects. In another aspect, the term "method for the treatment", as used herein, refers to "method to treat".

The terms "intermittent dosing regimen" or "intermittent dosing schedule", as used herein, mean a dosing regimen that comprises administering Compound 1, followed by a resting period. For example, Compound 1 is administered according to an intermittent dosing schedule of at least two cycles, each cycle comprising (a) a dosing period and thereafter (b) a resting period.

As used herein, the term "resting period" refers, in particular, to a period of time during which the patient is not given Compound 1 (i.e., a period of time wherein the treatment with Compound 1 is withheld). For example, if Compound 1 is given on a daily basis, there would be rest period if the daily administration is discontinued for some time, e.g., for some number of days, or the plasma concentration of Compound 1 is maintained at sub-therapeutic level for some time e.g., for some number of days. The dosing period and/or the dose of Compound 1 can be the same or different between cycles. The total treatment time (i.e., the number of cycles for treatment) may also vary from patient to patient based, for example, on the particular patient being treated (e.g., Stage I HD patient).

In another aspect, an intermittent dosing schedule comprises at least two cycles, each cycle comprising (a) a dosing period during which a therapeutically effective amount of Compound 1 is administered to said patient and thereafter (b) a resting period. The terms "intermittent dosing regimen" or "intermittent dosing schedule", as used herein, refer to both a dosing regimen for Compound 1 alone (i.e. monotherapy) or a dosing regimen for administering Compound 1 in combination with at least a further active ingredient (i.e. combination therapy). In another aspect, the terms "intermittent dosing regimen" or "intermittent dosing schedule" refers to repeated on/off treatment, wherein Compound 1 is administered at regular intervals in a periodic manner, for example, once a day, every 2 days, every 3 days, every 4 days, once a week, or twice a week.

The term "once a day" or "once daily" or "QD" in the context of administering a drug means herein administering one dose of a drug once each day, wherein the dose is, for example, administered on the same day of the week.

In one aspect, the terms "administering" or "administration of Compound 1 once a day," as used herein, refer to the amount of Compound 1 in the tablet in a range of from 1 mg to 100 mg, administered once a day.

In another aspect, the amount of Compound 1 in the tablet is in a range of from 1 mg to 200 mg, administered once a day.

In another aspect, the amount of Compound 1 in the tablet is in a range of from 1 mg to 100 mg, administered once a day.

In another aspect, the amount of Compound 1 in the tablet is selected from 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, and 200 mg, administered once a day.

In another aspect, the amount of Compound 1 in the tablet is selected from 1 mg, 5 mg, 10 mg, 15 mg, 20 mg, 25 mg, 30 mg, 35 mg, 50 mg, 60 mg, 65 mg, 70 mg, 75 mg, 80 mg, 85 mg, 90 mg, 95 mg, 100 mg, 110 mg, 120 mg, 135 mg, and 140 mg, administered once a day.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, 75 mg, 80 mg, 85 mg, 90 mg, 95 mg, 100 mg, 110 mg, 120 mg, 135 mg, and 140 mg, administered once a day.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, and 100 mg, administered once a day.

In another aspect, the amount of Compound 1 in the tablet is selected from 1 mg, 5 mg, 10 mg, 20 mg, 30 mg, and 50 mg, administered once a day.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, and 100 mg, administered once a day.

In another aspect, the amount of Compound 1 in the tablet is selected from 1 mg, 5 mg or 50 mg, administered once a day.

In another aspect, the amount of Compound 1 in the tablet is selected from 5 mg or 50 mg, administered once a day.

In another aspect, the amount of Compound 1 in the tablet is selected from 5 mg, 10 mg, 20 mg, and 30 mg, administered once a day.

In another aspect, the amount of Compound 1 in the tablet is selected from 5 mg, 10 mg, and 20 mg, administered once a day.

The term "once a week" or "once weekly" or "QW" in the context of administering Compound 1 means herein administering one dose of Compound 1 once each week, wherein the dose is, for example, administered on the same day each week.

In one aspect, the terms "administering" or "administration of Compound 1 once a week," as used herein, refer to Compound 1 administered in an amount selected from a range of from 25 mg to 100 mg once a week, a range of from 25 mg to 200 mg once a week, and a range of from 50 mg to 200 mg once a week.

In another aspect, Compound 1 is administered in an amount selected from 35 mg once a week, 70 mg once a week, and 140 mg once a week.

The term "twice a week" or "twice weekly" or "BIW' in the context of administering Compound 1 means herein administering one dose of Compound 1 twice each week, wherein each dose is administered on separate days each week at regular intervals in a range of from 48 to 72 hours.

In one aspect, the terms "administering" or "administration of Compound 1 twice a week," as used herein, refer to Compound 1 administered in an amount selected from a range of from 10 mg to 100 mg twice a week, a range of from 10 mg to 200 mg twice a week, and a range of from 25 mg to 100 mg twice a week.

In another aspect, Compound 1 is administered in an amount selected from a range of from 10 mg to 20 mg twice a week, such as about 15 mg twice a week, a range of from 30 mg to 40 mg twice a week, such as 35 mg twice a week, and a range of from 50 mg to 90 mg twice a week, such as 70 mg twice a week.

The term "about" in relation to a numerical value X means, for example, X $\pm$ 15%, including all the values within this range.

The terms "disease-modifying therapy" or disease-modifying treatment", as used herein, refer to a drug that can modify or change the course of a condition or a disorder or a disease (i.e. a disease-modifying drug), such as HD, as defined herein.

The term "subject", as used herein, refers to a mammalian organism, preferably a human being (male or female).

The term "patient", as used herein, refers to a subject who is diseased and would benefit from the treatment.

The term "a subject in need of', as used herein, refers to a treatment if such subject (patient) would benefit biologically, medically or in quality of life from such treatment.

The terms "a therapeutically effective amount" or "an effective amount" of Compound 1, as used herein, refer to an amount of Compound 1 that will elicit the biological or medical response of a subject. In another embodiment, the term refers to the amount of of Compound 1 that, when administered to a subject, is effective to at least partially ameliorate a condition, or a disorder or a disease.

The term "one or more" refers to either one or a number above one (e.g. 2, 3, 4, 5, etc.).

2. Compound

An active ingredient of the tablet compositions disclosed herein is 2-[3-(2,2,6,6-tetramethylpiperidin-4-yl)-3H-[1,2,3]triazolo[4,5-c]pyridazin-6-yl]-5-(2H-1,2,3-triazol-2-yl)phenol (Compound 1) or a pharmaceutically acceptable salt thereof. Compound 1 and a suitable method of making it are disclosed in WO2020/005873 (compound 163 in that publication).

In one aspect, the amount of Compound 1 in the tablet, by weight of the total weight of the tablet, is selected from 5% to 30%, 5% to 25%, 10% to 20%, and 10%.

In one aspect, the amount of Compound 1 in the tablet is in a range from 1 mg to 200 mg.

In another aspect, the amount of Compound 1 in the tablet is in a range from 1 mg to 100 mg.

In another aspect, the amount of Compound 1 in the tablet is selected from 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, and 200 mg.

In another aspect, the amount of Compound 1 in the tablet is selected from 1 mg, 5 mg, 10 mg, 15 mg, 20 mg, 25 mg, 30 mg, 35 mg, 50 mg, 60 mg, 65 mg, 70 mg, 75 mg, 80 mg, 85 mg, 90 mg, 95 mg, 100 mg, 110 mg, 120 mg, 135 mg, and 140 mg.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, 75 mg, 80 mg, 85 mg, 90 mg, 95 mg, 100 mg, 110 mg, 120 mg, 135 mg, and 140 mg.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, and 100 mg.

In another aspect, the amount of Compound 1 in the tablet is selected from 1 mg, 5 mg, 10 mg, 20 mg, 30 mg, and 50 mg.

In another aspect, the amount of Compound 1 in the tablet is selected from 1 mg, 5 mg, 10 mg, 15 mg, 20 mg, 25 mg, 30 mg, 50 mg, 60 mg, 65 mg, 70 mg, and 100 mg.

In another aspect, the amount of Compound 1 in the tablet is selected from 1 mg, 5 mg, 10 mg, 20 mg, 30 mg, and 50 mg.

In another aspect, the amount of Compound 1 in the tablet is selected from 1 mg, 5 mg or 50 mg.

In another aspect, the amount of Compound 1 in the tablet is selected from 5 mg or 50 mg.

In another aspect, the amount of Compound 1 in the tablet is selected from 5 mg, 10 mg, 20 mg, and 30 mg.

In another aspect, the amount of Compound 1 in the tablet is selected from 5 mg, 10 mg, and 20 mg.

Tablets of Compound 1 can be made by direct compression, by admixing Compound 1 with excipients and compressing them to form a tablet. Tablets of Compound 1 can also be made by other methods, including wet granulation or dry granulation. When granulation is used, Compound 1 could be an intergranular and/or extragranular ingredient of the tablet. In one aspect of the present invention, Compound 1 is an intragranular ingredient of the tablet. Compound 1 can be mixed with at least one intragranular excipient and wet or dry granulated to form an intragranular blend used in making a tablet. In an aspect of the present invention, a tablet is made by a process that includes mixing Compound 1 with at least one intragranular excipient and wet granulating the mixture to form an intragranular blend, mixing the intragranular blend with at least one extragranular excipient, and compressing the resulting mixture to form a tablet.

### 3. Excipients

In one aspect, the tablets provided herein comprise excipients selected from the group consisting of a diluent, a binder, a surfactant, a disintegrant, a glidant, and a lubricant. In the aspects of the tablets provided herein, some excipients are only intragranular excipients or extragranular excipients, while others are both intragranular and extragranular excipients.

### a. Diluent

Diluents are excipients which are used for diluting formulation components such as active ingredients and adjusting them to amounts appropriate to the formulation, and in some cases, for imparting stability or improved moldability. Examples of diluents include sugars such as lactose or glucose, sugar alcohols such as mannitol, xylitol, maltitol, sorbitol, isomalt and crystalline cellulose. lactose, glucose, sucrose, fructose, maltose, trehalose. Microcrystalline cellulose can also serve as a diluent in tablet formulations, and it is included as an intragranular excipient in the tablets of the invention. However, the term "diluent" as used herein refers to diluents other than microcrystalline cellulose.

In one aspect, the amount of microcrystalline cellulose included as an intragranular excipient in tablets of the present invention is selected from the group consisting of a range of from about 15% to about 25%, and about 15% to about 20%, by weight of the total weight of the tablet. In another aspect, the amount of microcrystalline cellulose included as an intragranular excipient in tablets of the present invention is about 20% by weight of the total weight of the tablet. In one aspect, the diluent included as an intragranular excipient is present in a ratio of microcrystalline cellulose to diluent selected from the group consisting of a ratio in a range of from about 1:1 to about 1:4, and about 1:1 to about 1:2. In another aspect, the diluent included as an intragranular excipient is present in a ratio of about 1:2. In one aspect, the amount of diluent included as an intragranular excipient is selected from the group consisting of a range of from about 15% to about 40%, and about 20% to about 40%, by weight of the total weight of the tablet. In another aspect, the amount of diluent included as an intragranular excipient is about 40% by weight of the total weight of the tablet. When the diluent is included as an extragranular excipient, in one aspect, it is present in an amount selected from the group consisting of a range of from about 5% to about 25%, and about 10% to about 25%, by weight of the total weight of the tablet. When the diluent is included as an extragranular excipient, in another aspect, it is present in an amount of about 20% by weight of the total weight of the tablet.

In one embodiment, the diluent is a lactose, preferably lactose monohydrate.

## b. Binder

Binders are classified as excipients which import the stickiness for maintaining quality after forming a tablet. The amount of binder in the tablets provided herein varies based upon, for example, the type of binders (properties such as molecular weight, solubility and viscosity), the type and amount of other excipients, the type and amount of the composite, and its dosage form of the formulation step (granulation method and tableting method).

Examples of binders that could be used include hydroxypropyl cellulose, hypromellose, methyl cellulose, hydroxyethyl cellulose, hydroxyethyl methylcellulose, hydroxypropyl starch, maize starch, peas starch, pregelatinized starch, acacia, tragacanth, modified gums, gelatin, and povidone.

In one aspect, povidone is included as an intragranular excipient a tablet of the invention, and is present in an amount of 1% to about 5%, alternatively, 1.5% to about 4%, alternatively about 2% to about 3%, alternatively about 2%, by weight of the total weight of the tablet.

## c. Disintegrant

Disintegrants are excipients which function to disintegrate a tablet by absorbing water after administration, swelling, and thereby facilitating release of the active ingredient. In an aspect of the present tablet, a disintegrant is included as both an intragranular and as an extragranular excipient. In one aspect, the amount of disintegrant is selected from the group consisting of a range of from about 1% to about 5%, about 1% to about 3%, and about 1.5% to about 2.5% by weight of the total weight of the tablet in each of the intragranular and extragranular parts of the tablet. In another aspect, the amount of disintegrant is about 2.5% by weight of the total weight of the tablet in each of the intragranular and extragranular parts of the tablet.

Examples of suitable disintegrants include sodium starch glycolate, Crospovidone, crosslinked alginic acid, crosslinked starch, crosslinked alginate sodium, carmellose, carmellose calcium, croscarmellose sodium, glycerin fatty acid ester, low-substituted sodium carboxymethyl starch and partially pregelatinized starch. In one embodiment, the disintegrant is carmellose sodium.

## d. Surfactant

A surfactant is an excipient used to improve solubilization of the active agent. A surfactant can be included as an intragranular and/or extragranular excipient. In one embodiment a surfactant is included as an intragranular excipient and in another it is included as an extragranular excipient.

Non-limiting examples of surfactants that can be used include quaternary ammonium compounds, for example dioctyl sodium sulfosuccinate, polyoxyethylene alklphenyl ethers, for example nonoxynol 9, nonoxynol 10 and octoxynol 9, poloxamers (poloxyethylene and polyoxypropylene block copolymers, such as Poloxamer 407), polyoxyethylene fatty acid glycerides and oils, for example polyoxyethylene (8), caprylic/capric mono- and diglycerides, polyoxyethylene (35) castor oil and polyoxyethylene (40) hydrogenated castor oil, polyethylene alkyl ethers, for example poloxyethylene (20) cetostearyl ether, polyoxyethelene fatty acid esters, for example polyoxyethylene (40) stearate, polyoxyethylene sorbitan esters, for example polysorbate 20 and polysorbate 80 (e.g. Tween 80), propylene glycol fatty esters, for example propylene glycol laurate, sodium lauryl sulfate, fatty acids and salts thereof, for example oleic acid, sodium oleate and triethanolamine oleate, glyceryl fatty acid esters, for example sorbitan monolaurate, sorbitan monooleate, sorbitan monopalmitate and sorbitan monostearate, tyloxapol, and mixtures thereof.

In one aspect, the amount of surfactant included in the tablet is suitably selected from the group consisting of a range of from about 0.5% to about 2%, and about 0.5% to about 1.5%. In another aspect, the amount of surfactant included in the tablet is preferably about 1%.

The surfactant included in the present tablet is a poloxamer, preferably Poloxamer 407.

## e. Lubricant

Lubricants are excipients that can be used to reduce friction between equipment and a granulated mixture during compression to form a tablet. Examples of suitable lubricants include, either individually or in combination, glyceryl behenate, glyceryl behaptate; sodium stearyl fumarate, stearic acid and salts thereof, including magnesium, calcium and sodium stearates; hydrogenated vegetable oils; colloidal silica; talc; waxes; boric acid; sodium benzoate; sodium acetate; sodium fumarate; sodium chloride; DL-leucine; polyethylene glycol; sodium oleate; sodium lauryl sulfate; and magnesium lauryl sulfate. When a lubricant is used in the tablet of the invention, it is preferably magnesium stearate.

In one aspect, lubricant is included as an extragranular excipient by weight of the total weight of the tablet. In another aspect, the amount of lubricant included as an extragranular excipient is selected from the group consisting of a range of from 0.5% to about 3%, and about 1% to about 2% by weight of the total weight of the tablet. In another aspect, the amount of lubricant included as an extragranular excipient is about 1.5% by weight of the total weight of the tablet. The lubricant is preferably magnesium stearate.

### f. Glidant

A glidant is an excipient used as an anti-adherent. Examples of glidants include colloidal silicon dioxide, hydrated sodium sulfoaluminate and talc. In one aspect of the present tablet, a glidant is included as an extragranular excipient. When present, the glidant is present in an amount selected from the group consisting of a range of from about 0.25% to about 2%, and about 0.25% to about 1%, by weight of the total weight of the tablet. When present, the glidant is present in an amount of about 0.5% by weight of the total weight of the tablet.

### g. Other Excipients

The tablet provided herein may contain various excipients other than the ones described above. Examples of other excipients include, but are not limited to solubility enhancers, stabilizers, pH adjustors, coating agents and pigments.

### 4. Coating

The tablets of the invention are optionally coated with a film suitable for an immediate release tablet, such as a film coating comprising polyvinyl alcohol and hydroxypropyl methyl cellulose.

### 5. Process of Making Tablet

In one aspect, the invention is a process for making a tablet of the invention, using wet granulation. That is suitably done in three stages according to the following steps:

Stage one (intragranular stage):

(a) Dissolving the povidone in water,

(b) Passing the remaining intragranular ingredients through a sieve, e.g. a #30 mesh,

(c) Blending the sieved ingredients to form a granulate,

(d) Wetting the granulate with the povidone solution and blending until optimum granules are obtained,

(e) Drying the optimum granules, preferably until a moisture content of about 2% is achieved,

(f) Passing the dried granules through a sieve of a particular size, e.g. a #20 mesh sieve.

Stage two (extragranular stage)

(a) Passing all the extragranular excipients except for the lubricant (e.g. magnesium stearate) through a sieve, e.g. a #20 mesh,

(b) Adding the sieved extragranular excipients to the milled granules from stage one and blending,

(c) Sieving the lubricant, e.g. using a #30 mesh sieve, and adding it to the blend and blending further,

Stage three (tableting)

Compressing the blend from the final step of Stage two above into tablets using a tablet press, and optionally coating each tablet with a film coating.

### 6. Tablet Characteristics

Tablets of Compound 1 of the present invention preferably have all of the following characteristics:

- Rapid disintegration when dissolved in 0.01 N HCl

- Good bioavailability of Compound 1 when administered to a subject

- Physical integrity of the tablet, e.g. good friability, and strength.

- Stability of Compound 1 in the tablet.

## 7. Use of Compound 1

The tablets of Compound 1 provided herein are useful for treating or ameliorating Huntington's Disease.

In one aspect, treating or ameliorating Huntington's Disease with Compound 1, or a pharmaceutically acceptable salt thereof, as a disease-modifying therapy, results from the production of an in-frame stop codon between exons 49 and 50 in the HTT mRNA transcript; wherein, the resulting decrease in mRNA and reduction of wildtype and mutant HTT protein has one or more of the following effects:

(i) slowed rate of decline in loss of motor function associated with Huntington's disease, wherein the slowed rate of decline of motor function associated with Huntington's disease after treatment with Compound 1 is shown by decrease in mRNA and reduction of wildtype and mutant HTT protein or by comparison to placebo; wherein, motor function is selected from the group consisting of ocular motor function, dysarthria, dystonia, chorea, postural stability and gait; and, is assessed by using standard clinical scales, such as the UHDRS motor assessment scale (e.g. in Movement Disorders, 1996, 11, 136-142);

(ii) slowed rate of cognitive decline associated with Huntington's disease, wherein the slowed rate of cognitive decline associated with Huntington's disease after treatment with Compound 1 is shown by decrease in mRNA and reduction of wildtype and mutant HTT protein or by comparison to placebo; wherein, cognitive function is selected from the group consisting of attention, processing speed, visuospatial processing, timing, emotion processing, memory, verbal fluency, psychomotor function, and executive function; and, is assessed by using standard clinical scales, such as the Symbol Digit Modalities Test, the Stroop Word Reading Test, the Montreal Cognitive Assessment or the HD Cognitive Assessment Battery (comprising the Symbol Digit Modalities Test, Trail Making Test B, One Touch Stockings, Paced Tapping, Emotion Recognition Test, Hopkins Verbal Learning Test); e.g. in Movement Disorders, 2014, 29 (10), 1281-1288];

(iii) slowed rate of psychiatric decline associated with Huntington's disease, wherein the slowed rate of psychiatric decline associated with Huntington's disease after treatment with Compound 1 is shown by decrease in mRNA and reduction of wildtype and mutant HTT protein or by comparison to placebo; wherein, psychiatric decline is selected from the group consisting of apathy, anxiety, depression, obsessive compulsive behavior, suicidal thoughts, irritability and agitation; and, is assessed by using standard clinical scales, such as the Apathy Evaluation Scale or by the Hospital Anxiety and Depression Scale; e.g. in Movement Disorders, 2016, 31 (10), 1466-1478, Movement Disorders, 2015, 30 (14), 1954-1960];

(iv) slowed rate of decline of functional capacity associated with Huntington's disease, wherein the slowed rate of decline of functional capacity associated with Huntington's disease after treatment with Compound 1 is shown by decrease in mRNA and reduction of wildtype and mutant HTT protein or by comparison to placebo; wherein, functional capacity is selected from the group consisting of capacity to work, capacity to handle financial affairs, capacity to manage domestic chores, capacity to perform activities of daily living, and level of care needed; and, is assessed by using standard clinical scales, such as the UHDRS Total Functional Capacity, Functional Assessment and Independence scales (e.g. in Movement Disorders, 1996, 11, 136-142);

(v) slowed progression of Huntington's disease pathophysiology, wherein the slowed progression of Huntington's disease pathophysiology associated with Huntington's disease [e.g. reducing the rate of brain (e.g. whole brain, caudate, striatum or cortex) volume loss (e.g. % from baseline volume)] after treatment with Compound 1 is shown by decrease in mRNA and reduction of wildtype and mutant HTT protein or by comparison to placebo, and assessed using standard techniques, such as MRI (e.g. by neuroimaging measures)(see, .e.g. Lancet Neural. 2013, 12 (7), 637-649);

(vi) slowed onset of Huntington's disease or the onset of symptoms associated with Huntington's disease, wherein the slowed onset of Huntington's disease or the onset of symptoms associated with Huntington's disease after treatment with Compound 1 is shown by decrease in mRNA and reduction of wildtype and mutant HTT protein or by comparison to placebo, and assessed by using standard clinical scales, such as the Huntington's Disease Health-related Quality of Life questionnaire (HDQoL) (e.g. in Movement Disorders, 2018, 33 (5), 742-749); or,

(vii) reduced decline in quality of life associated with Huntington's disease, wherein the slowed onset of Huntington's disease or the onset of symptoms associated with Huntington's disease after treatment with Compound 1 is shown by decrease in mRNA and reduction of wildtype and mutant HTT protein or by comparison to placebo, and is assessed by using standard clinical scales, such as the Huntington's Disease Health-related Quality of Life questionnaire (HDQoL) (e.g. in Movement Disorders, 2018, 33 (5), 742-749).

In another aspect, treating or ameliorating Huntington's Disease with Compound 1, or a pharmaceutically acceptable salt thereof, has one or more of the following effects: (i) a favorable therapeutic profile, such as a favorable safety profile or metabolic profile; or, (ii) a favorable offtarget effect profile, such as a favorable psychiatric adverse event profile, a favorable toxicity (e.g. genotoxicity) or cardiovascular adverse event (e.g. blood pressure, heart rate, electrocardiography parameters) profile.

In one aspect, a patient in need thereof is orally administered a tablet of the invention, containing a therapeutically effective amount of Compound 1.

In another aspect, the tablet contains the therapeutically effective amount in a range of from 1 mg to 200 mg of Compound 1.

In another aspect, the tablet contains the therapeutically effective amount in a range of from 1 mg to 100 mg of Compound 1.

In another aspect, the tablet contains the therapeutically effective amount selected from 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, and 200 mg.

In another aspect, the tablet contains the therapeutically effective amount selected from 1 mg, 5 mg, 10 mg, 15 mg, 20 mg, 25 mg, 30 mg, 35 mg, 50 mg, 60 mg, 65 mg, 70 mg, 75 mg, 80 mg, 85 mg, 90 mg, 95 mg, 100 mg, 110 mg, 120 mg, 135 mg, and 140 mg.

In another aspect, the tablet contains the therapeutically effective amount selected from 1 mg, 5 mg, 10 mg, 15 mg, 20 mg, 25 mg, 30 mg, 50 mg, 60 mg, 65 mg, 70 mg, 75 mg, 80 mg, 85 mg, 90 mg, 95 mg, 100 mg, 110 mg, 120 mg, 135 mg, and 140 mg.

In another aspect, the tablet contains the therapeutically effective amount selected from 1 mg, 5 mg, 10 mg, 15 mg, 20 mg, 25 mg, 30 mg, 50 mg, 60 mg, 65 mg, 70 mg, and 100 mg.

In another aspect, the tablet contains the therapeutically effective amount selected from 1 mg, 5 mg, 10 mg, 20 mg, 30 mg, and 50 mg.

In another aspect, the tablet contains the therapeutically effective amount selected from 1 mg, 5 mg, 10 mg, 15 mg, 20 mg, 25 mg, 30 mg, 50 mg, 60 mg, 65 mg, 70 mg, and 100 mg.

In another aspect, the tablet contains the therapeutically effective amount selected from 1 mg, 5 mg or 50 mg.

In another aspect, the tablet contains the therapeutically effective amount selected from 5 mg or 50 mg.

In another aspect, the tablet contains the therapeutically effective amount selected from 5 mg, 10 mg, 20 mg, and 30 mg.

In another aspect, the tablet contains the therapeutically effective amount selected from 5 mg, 10 mg, and 20 mg.

In one aspect, a patient in need thereof is orally administered a tablet of the invention, containing a therapeutically effective amount of Compound 1, administered once a day.

In another aspect, the tablet contains the therapeutically effective amount in a range of from 1 mg to 200 mg of Compound 1, administered once a day.

In another aspect, the tablet contains the therapeutically effective amount in a range of from 1 mg to 100 mg of Compound 1, administered once a day.

In another aspect, the tablet contains the therapeutically effective amount selected from 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, and 200 mg, administered once a day.

In another aspect, the tablet contains the therapeutically effective amount selected from 1 mg, 5 mg, 10 mg, 15 mg, 20 mg, 25 mg, 30 mg, 35 mg, 50 mg, 60 mg, 65 mg, 70 mg, 75 mg, 80 mg, 85 mg, 90 mg, 95 mg, 100 mg, 110 mg, 120 mg, 135 mg, and 140 mg, administered once a day.

In another aspect, the tablet contains the therapeutically effective amount selected from 1 mg, 5 mg, 10 mg, 15 mg, 20 mg, 25 mg, 30 mg, 50 mg, 60 mg, 65 mg, 70 mg, 75 mg, 80 mg, 85 mg, 90 mg, 95 mg, 100 mg, 110 mg, 120 mg, 135 mg, and 140 mg, administered once a day.

In another aspect, the tablet contains the therapeutically effective amount selected from 1 mg, 5 mg, 10 mg, 15 mg, 20 mg, 25 mg, 30 mg, 50 mg, 60 mg, 65 mg, 70 mg, and 100 mg, administered once a day.

In another aspect, the tablet contains the therapeutically effective amount selected from 1 mg, 5 mg, 10 mg, 20 mg, 30 mg, and 50 mg, administered once a day.

In another aspect, the tablet contains the therapeutically effective amount selected from 1 mg, 5 mg, 10 mg, 15 mg, 20 mg, 25 mg, 30 mg, 50 mg, 60 mg, 65 mg, 70 mg, and 100 mg, administered once a day.

In another aspect, the tablet contains the therapeutically effective amount selected from 1 mg, 5 mg or 50 mg, administered once a day.

In another aspect, the tablet contains the therapeutically effective amount selected from 5 mg or 50 mg, administered once a day.

In another aspect, the tablet contains the therapeutically effective amount selected from 5 mg, 10 mg, 20 mg, and 30 mg, administered once a day.

In another aspect, the tablet contains the therapeutically effective amount selected from 5 mg, 10 mg, and 20 mg, administered once a day.

In another aspect, the tablet contains the therapeutically effective amount of 1 mg of Compound 1.

In another aspect, the tablet contains the therapeutically effective amount of 5 mg of Compound 1.

In another aspect, the tablet contains the therapeutically effective amount of 10 mg of Compound 1.

In another aspect, the tablet contains the therapeutically effective amount of 15 mg of Compound 1.

In another aspect, the tablet contains the therapeutically effective amount of 20 mg of Compound 1.

In another aspect, the tablet contains the therapeutically effective amount of 25 mg of Compound 1.

In another aspect, the tablet contains the therapeutically effective amount of 30 mg of Compound 1.

In another aspect, the tablet contains the therapeutically effective amount of 35 mg of Compound 1.

In another aspect, the tablet contains the therapeutically effective amount of 40 mg of Compound 1.

In another aspect, the tablet contains the therapeutically effective amount of 45 mg of Compound 1.

In another aspect, the tablet the therapeutically effective amount of contains 50 mg of Compound 1.

In another aspect, the tablet contains the therapeutically effective amount of 55 mg of Compound 1.

In another aspect, the tablet contains the therapeutically effective amount of 60 mg of Compound 1.

In another aspect, the tablet contains the therapeutically effective amount of 65 mg of Compound 1.

In another aspect, the tablet contains the therapeutically effective amount of 70 mg of Compound 1.

In another aspect, the tablet contains the therapeutically effective amount of 75 mg of Compound 1.

In another aspect, the tablet contains the therapeutically effective amount of 80 mg of Compound 1.

In another aspect, the tablet contains the therapeutically effective amount of 85 mg of Compound 1.

In another aspect, the tablet contains the therapeutically effective amount of 90 mg of Compound 1.

In another aspect, the tablet contains the therapeutically effective amount of 95 mg of Compound 1.

In another aspect, the tablet contains the therapeutically effective amount of 100 mg of Compound 1.

In another aspect, the tablet contains the therapeutically effective amount of 105 mg of Compound 1.

In another aspect, the tablet contains the therapeutically effective amount of 110 mg of Compound 1.

In another aspect, the tablet contains the therapeutically effective amount of 115 mg of Compound 1.

In another aspect, the tablet contains the therapeutically effective amount of 120 mg of Compound 1.

In another aspect, the tablet contains the therapeutically effective amount of 125 mg of Compound 1.

In another aspect, the tablet contains the therapeutically effective amount of 130 mg of Compound 1.

In another aspect, the tablet contains the therapeutically effective amount of 135 mg of Compound 1.

In another aspect, the tablet contains the therapeutically effective amount of 140 mg of Compound 1.

In another aspect, the tablet contains the therapeutically effective amount of 145 mg of Compound 1.

In another aspect, the tablet the therapeutically effective amount of contains 150 mg of Compound 1.

In another aspect, the tablet contains the therapeutically effective amount of 155 mg of Compound 1.

In another aspect, the tablet contains the therapeutically effective amount of 160 mg of Compound 1.

In another aspect, the tablet contains the therapeutically effective amount of 165 mg of Compound 1.

In another aspect, the tablet contains the therapeutically effective amount of 170 mg of Compound 1.

In another aspect, the tablet contains the therapeutically effective amount of 175 mg of Compound 1.

In another aspect, the tablet contains the therapeutically effective amount of 180 mg of Compound 1.

In another aspect, the tablet contains the therapeutically effective amount of 185 mg of Compound 1.

In another aspect, the tablet contains the therapeutically effective amount of 190 mg of Compound 1.

In another aspect, the tablet contains the therapeutically effective amount of 195 mg of Compound 1.

In another aspect, the tablet contains the therapeutically effective amount of 200 mg of Compound 1.

In another aspect, a tablet containing the therapeutically effective amount of Compound 1 is administered once per day.

In another aspect, the tablet containing the therapeutically effective amount of Compound 1 is administered twice per day.

In another aspect, a tablet containing the therapeutically effective amount of Compound 1 is administered three times per day.

In another aspect, a tablet containing the therapeutically effective amount of Compound 1 is administered once per week.

In another aspect, a tablet containing the therapeutically effective amount of Compound 1 is administered once every two weeks.

In one aspect, a use of a tablet containing a therapeutically effective amount of Compound 1, or a pharmaceutically acceptable salt thereof, in treating or ameliorating Huntington's Disease as a disease-modifying therapy, includes Huntington's disease selected from the group consisting of Huntington's Disease genetically characterized by CAG repeat expansion of from 36 to 39 in the *HTT* gene on chromosome 4; and, Huntington's disease genetically characterized by CAG repeat expansion of from >39 in the *HTT* gene on chromosome 4.

In one aspect, a use of a tablet containing a therapeutically effective amount of Compound 1, or a pharmaceutically acceptable salt thereof, in treating or ameliorating Huntington's Disease as a disease-modifying therapy, includes Huntington's disease selected from the group consisting of manifest Huntington's disease, juvenile Huntington's disease, pediatric Huntington's disease, early stage of Huntington's disease, middle stage of Huntington's disease, advanced stage of Huntington's disease, stage I of Huntington's disease, stage II of Huntington's disease, stage III of Huntington's disease, stage IV of Huntington's disease, stage V of Huntington's disease, and pre-manifest Huntington's disease.

In one aspect, a tablet containing a therapeutically effective amount of Compound 1, or a pharmaceutically acceptable salt thereof, is administered according to an intermittent dosing schedule.

In another aspect, a tablet containing a therapeutically effective amount of Compound 1, or a pharmaceutically acceptable salt thereof, is administered once a week or twice a week.

In another aspect, a tablet containing a therapeutically effective amount of Compound 1, or a pharmaceutically acceptable salt thereof, is administered orally.

In another aspect, a tablet containing a therapeutically effective amount of Compound 1, or a pharmaceutically acceptable salt thereof, is provided in the form of a pharmaceutical composition.

In another aspect, a tablet containing a therapeutically effective amount of Compound 1, or a pharmaceutically acceptable salt thereof, is provided in the form of a pharmaceutical combination.

In another aspect, a tablet containing a therapeutically effective amount of Compound 1, or a pharmaceutically acceptable salt thereof, is administered following gene therapy or treatment with an antisense compound.

In one aspect, a method of treatment for slowing progression of Huntington's disease in a subject in need thereof, comprising administering to the subject one or more tablets containing a therapeutically effective amount of Compound 1.

In another aspect, a method of treatment for slowing the decline of motor function associated with Huntington's disease in a subject in need thereof, comprising administering to the subject one or more tablets containing a therapeutically effective amount of Compound 1.

In another aspect, a method of treatment for slowing cognitive decline associated with Huntington's disease in a subject in need thereof, comprising administering to the subject one or more tablets containing a therapeutically effective amount of Compound 1.

In another aspect, a method of treatment for slowing psychiatric decline associated with Huntington's disease in a subject in need thereof, comprising administering to said subject one or more tablets containing a therapeutically effective amount of Compound 1.

In another aspect, a method of treatment for slowing the decline of functional capacity associated with Huntington's disease in a subject in need thereof, comprising administering to the subject one or more tablets containing a therapeutically effective amount of Compound 1.

In another aspect, a method of treatment for slowing the progression of Huntington's disease pathophysiology [e.g. reducing the rate of brain (e.g. whole brain, caudate, striatum or cortex) volume loss (e.g. % from baseline volume)] associated with Huntington's disease (e.g. as assessed by MRI)] in a subject in need thereof, comprising administering to the subject one or more tablets containing a therapeutically effective amount of Compound 1.

In another aspect, a method of treatment for slowing the decline of motor function associated with Huntington's disease in a subject in need thereof, comprising administering to the subject one or more tablets containing a therapeutically effective amount of Compound 1; wherein, motor function is selected from the group consisting of ocular motor function, dysarthria, dystonia, chorea, postural stability and gait.

In another aspect, a method of treatment for slowing cognitive decline associated with Huntington's disease in a subject in need thereof, comprising administering to the subject one or more tablets containing a therapeutically effective amount of Compound 1; wherein, cognitive decline is selected from the group consisting of attention, processing speed, visuospatial processing, timing, emotion processing, memory, verbal fluency, psychomotor function, and executive function.

In another aspect, a method of treatment for slowing psychiatric decline associated with Huntington's disease in a subject in need thereof, comprising administering to said subject one or more tablets containing a therapeutically effective amount of Compound 1; wherein, psychiatric decline is selected from the group consisting of apathy, anxiety, depression, obsessive compulsive behavior, suicidal thoughts, irritability and agitation.

In another aspect, a method of treatment for slowing the decline of functional capacity associated with Huntington's disease in a subject in need thereof, comprising administering to the subject one or more tablets containing a therapeutically effective amount of Compound 1; wherein, functional capacity comprises one or more selected from the group consisting of capacity to work, capacity to handle financial affairs, capacity to manage domestic chores, capacity to perform activities of daily living, and level of care needed.

In another aspect, a method of treatment for slowing the progression of Huntington's disease pathophysiology [e.g. reducing the rate of brain (e.g. whole brain, caudate, striatum or cortex) volume loss (e.g. % from baseline volume)] associated with Huntington's disease (e.g. as assessed by MRI)] in a subject in need thereof, comprising administering to the subject one or more tablets containing a therapeutically effective amount of Compound 1.

In one aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of Compound 1 in a range of from 1 to 200 mg.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of Compound 1 in a range of from 1 to 100 mg.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 1 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 5 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 10 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 15 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 20 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 25 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 30 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 35 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 40 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 45 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of contains 50 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 55 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 60 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 65 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 70 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 75 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 80 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 85 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 90 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 95 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 100 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 105 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 110 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 115 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 120 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 125 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 130 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 135 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 140 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 145 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 150 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 155 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 160 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 165 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 170 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 175 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 180 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 185 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 190 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 195 mg of Compound 1.

In another aspect, a method of treating or ameliorating Huntington's Disease in a subject in need thereof, comprising administering to the subject one or more tablets containing a therapeutically effective amount of Compound 1, or a pharmaceutically acceptable salt thereof, wherein each tablet contains a therapeutically effective amount of 200 mg of Compound 1.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, once per day.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, twice per day.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, three times per day.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, once per week.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, once every two weeks.

In one aspect, a method of treating or ameliorating Huntington's Disease in a subject in need thereof, comprises administering to the subject one or more tablets containing a therapeutically effective amount of Compound 1, or a pharmaceutically acceptable salt thereof, wherein Huntington's disease is selected from the group consisting of Huntington's Disease genetically characterized by CAG repeat expansion of from 36 to 39 in the *HTT* gene on chromosome 4; and, Huntington's disease genetically characterized by CAG repeat expansion of from >39 in the *HTT* gene on chromosome 4.

In one aspect, a method of treating or ameliorating Huntington's Disease in a subject in need thereof, comprises administering to the subject one or more tablets containing a therapeutically effective amount of Compound 1, or a pharmaceutically acceptable salt thereof, wherein Huntington's disease is selected from the group consisting of manifest Huntington's disease, juvenile Huntington's disease, pediatric Huntington's disease, early stage of Huntington's disease, middle stage of Huntington's disease, advanced stage of Huntington's disease, stage I of Huntington's disease, stage II of Huntington's disease, stage III of Huntington's disease, stage IV of Huntington's disease, stage V of Huntington's disease, and pre-manifest Huntington's disease.

In one aspect, a method of treating or ameliorating Huntington's Disease in a subject in need thereof, comprises administering to the subject one or more tablets containing a therapeutically effective amount of Compound 1, or a pharmaceutically acceptable salt thereof, according to an intermittent dosing schedule.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, once a day, once a week or twice a week.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, orally.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, in the form of a pharmaceutical composition.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, in the form of a pharmaceutical combination.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, following gene therapy or treatment with an antisense compound.

In another aspect, a method of treating or ameliorating Huntington's Disease in a subject in need thereof, comprises administering to the subject one or more tablets containing a therapeutically effective amount of Compound 1, or a pharmaceutically acceptable salt thereof, to produce an in-frame stop codon between exons 49 and 50 in the HTT mRNA.

In another aspect, a method of slowing progression of Huntington's Disease in a subject in need thereof, comprises administering to the subject one or more tablets containing a therapeutically effective amount of Compound 1, or a pharmaceutically acceptable salt thereof, to produce an in-frame stop codon between exons 49 and 50 in the HTT mRNA.

## EXAMPLES

The following examples include illustrations of aspects of the invention. The examples are not to be construed as limitations.

### Example 1

Six development batches (1-6) of tablets of Compound 1 were prepared using direct compression, by weighing and sieving components through mesh #35 followed by low shear mixing and compressing into tablets. However, the flow was not adequate and sticking to tablet punches was observed while tableting. Two batches of placebo tablets (7 and 8) were prepared the same way without Compound 1. The compositions of the baches are illustrated in Table 1, below.

TABLE 1

| Blend # | 1 | 2 | 3 | 4 | 5 | 6 |
|---|---|---|---|---|---|---|
| Excipient | %w/w | %w/w | %w/w | %w/w | %w/w | %w/w |
| Compound 1 | 5 | 5 | 2 | 10 | 2 | 10 |
| Lactose monohydrate | 38 | | 78 | 70 | 50 | 50 |
| Pearlitol® SD100 | | 38 | | | | |
| Pregelatinized starch STARX1500 | | | 18 | 18 | | |
| Microcrystalline cellulose | 50 | 50 | | | 41 | 33 |
| Sodium Starch Glycolate | 5 | 5 | | | | |
| Croscarmellose Sodium | | | | | 5 | 5 |
| Colloidal Silicon Dioxide | 1 | 1 | 1 | 1 | 1 | 1 |
| Magnesium Stearate | 1 | 1 | 1 | 1 | 1 | 1 |
| Total | 100 | 100 | 100 | 100 | 100 | 100 |

### Example 2

To resolve poor flow and sticking issues with direct compression found in the batches in Example 1, dry granulation using roller compaction was introduced to prepare another three batches (9, 10, and 11) by weighing and sieving the components through mesh #35, mixing with a turbula mixer followed by roller compaction. That was followed by crushing the ribbons and passing through mesh #20 and using a turbula mixer to mix the extra granular components, followed by compression in a tablet press. Good blend uniformity was obtained. The composition of all three batches was the same and similar to batch 6, above, except that it contained equal percent by weight of lactose monohydrate and mcc (41.5% each). However, different roller compaction parameters (roll speed, screw speed, and pressure) were used for each batch. Batch 9 produced the best ribbons on roller compaction. Batch 11 stuck to the roll and the ribbons were brittle, while batch 12 produced a discontinuous ribbon.

Dissolution tests were conducted on dry granulation batch 9, but there were granules floating on the surface of the dissolution media due to a wetting issue with Compound 1.

### Example 3

To mitigate the wetting issue observed in Example 2 a decision was made to introduce surfactants into the formulations. Direct compression batches 13 and 14 were prepared with 5% w/w sodium lauryl sulfate (SLS) and 1% Poloxamer 188, respectively, and with 50 mg strength of Compound 1. Batch 13 with SLS showed even more undissolved granules of Compound 1 floating around with minimal drug release than Batch 9, tested in Example 2. Batch 14 with 1% Poloxamer 188 showed better dissolution performance with no granules floating on the surface of the dissolution media. Thus, inclusion of Poloxamer seemed to mitigate the wetting of the Compound 1 granules. However, a mounding phenomenon observed during the dissolution experiment at 50 revolutions per minute (rpm), with complete drug release only observed when the paddle speed was increased to 150 rpm at 75 minutes.

A direct compression batch 17 was also prepared in a similar way with a lower concentration of microcrystalline cellulose and Poloxamer 407 as a surfactant. There were issues with poor processability with this batch.

The composition of batches 13, 14, and 17 are summarized in Table 2, below:

**TABLE 2**

| Blend # | 13 | 14 | 17 |
|---|---|---|---|
| **Excipient** | %w/w | %w/w | %w/w |
| **Compound 1** | 10 | 10 | 10 |
| **Lactose monohydrate** | 39 | 41 | 60 |
| **Pregelatinized starch STARX1500** | | | 10 |
| **Microcrystalline cellulose** | 39 | 41 | 13 |
| **Croscarmellose sodium** | 5 | 5 | 4 |
| **Sodium Lauryl Sulfate** | 5 | | |
| **Poloxamer 188** | | 1 | |
| **Poloxamer 407** | | | 1 |
| **Colloidal silicon dioxide** | 1 | 1 | 1 |
| **Magnesium stearate** | 1 | 1 | 1 |
| **Total** | 100 | 100 | 100 |

### Example 4

To minimize the mounding phenomenon in the dissolution vessel, wet granulation batches 15 and 16 were prepared with a lower amount of Avicel PH102 and 0.5% w/w and 2.5% w/w polyvinylpyrrolidone (PVP) K30, respectively and 1% Poloxamer 407. Wet granulation was performed using a mortar and pestle. Intragranular ingredients were passed through #20 mesh sieve and blended. Povidone K30 was dissolved in water to obtain granulation fluid. Then, the preblend was wet granulated with the povidone K30 solution using the mortar and pestle to obtain optimum granules. The wet mass was dried in a tray oven at 60oC until achieving a moisture content of about 2%. The dried granules were passed through #20 sieve and blended with #20 mesh screened extragranular excipients. The unlubricated blend was mixed with #35 mesh screened magnesium stearate to obtain the final blend.

Batch 15 was found to be an optimal formulation with little mounding upon dissolution at 50 rpm. Batch 16 with 2.5% w/w PVP K30 was found to be inferior to Batch 15 in terms of dissolution performance, most likely due to more compact granules due to a higher level of PVP K30 binder.

A wet granulation batch 18 was also prepared in the same way as above with a lower amount of Avicel PH102 (10% w/w) to test whether the mounding in dissolution could be reduced further. However, upon dissolution testing, that batch failed to release Compound 1 completely.

A wet granulation batch 19 was also prepared in a similar way as described above but with 30% mcc in both the intragranular and extragranular blends. This batch also had issues with mounding and poor integrity.

### Example 5

An additional dry granulation batch 20 was prepared with 41% microcrystalline cellulose (mcc) and lactose monohydrate in the intragranular blend but no mcc or lactose monohydrate in the extragranular components.

The composition of batches 15, 16, 18, 19, and 20 are shown in Table 3, below.

**TABLE 3**

| Blend # | 15 | 16 | 18 | 19 | 20 |
|---|---|---|---|---|---|
| **Excipient** | %w/w | %w/w | %w/w | %w/w | %w/w |
| **Intragranular Components** | | | | | |
| **Compound 1** | 10 | 10 | 10 | 10 | 10 |
| **Lactose monohydrate, FlowLac90** | 20 | 20 | 10 | 30 | 41 |
| **Microcrystalline Cellulose** | 20 | 20 | 10 | 30 | 41 |

(continued)

| Blend # | 15 | 16 | 18 | 19 | 20 |
|---|---|---|---|---|---|
| Excipient | %w/w | %w/w | %w/w | %w/w | %w/w |
| Povidone K30 | 1 | 2.5 | 1 | 1 | |
| Croscarmellose Sodium | 2.5 | 2.5 | 2 | 2 | 3 |
| Colloidal Silicon Dioxide | | | | | 0.5 |
| Magnesium Stearate | | | | | 0.5 |
| Extragranular Components | | | | | |
| Lactose monohydrate, FlowLac90 | 41.5 | 50 | 61.5 | 11.5 | |
| Croscarmellose Sodium | 2.5 | 2.5 | 3 | 3 | 2 |
| Poloxamer 407 micro Kolliphor® P407 micro | 1 | 1 | 1 | 1 | 1 |
| Colloidal Silicon Dioxide | 0.5 | 0.5 | 0.5 | 0.5 | 0.5 |
| Magnesium Stearate | 1 | 1 | 1 | 1 | 0.5 |
| Total | | | | | |

**Example 6**

Dissolution performance of batches 9, 14, 15, 16, 17, 18, and 20 were tested in 500 ml 0.01N HCl while stirring with paddles at 50 rpm to 60 min, increased to 75 rpm to 75 min, increased to 150 rpm until 90 min, removing 5 ml at 5, 10, 15, 20, 30, 45, 60, 75, and 90 min.

Batches 15, 16, and 17 were also tested for degradation at 7 and 14 days after storage at 80oC at 5% relative humidity and at 80°C and 75% relative humidity. No degradation was found in any of the samples at the lower humidity, and degradation was minimal and comparable in all three batches tested at the higher humidity level.

Dissolution stability of Batch 15 was tested at various paddle speeds (50, 65, and 75 revolutions per minute) and at accelerated temperature conditions. It was found that the % of drug released at each time point was higher at faster speeds, and 81%, 88%, and 95% of the initial release respectively was released at each speed, respectively in the first 5 minutes. Release rates were even faster when tested in 0.01N HCl at a paddle speed of 75 rpm at room temperature, 40°C, or 65°C, where the % of initial release was 95.4, 92.6, and 96.4, respectively.

Based on the results above, Batches 15 (wet granulation) and 20 (dry granulation) were selected for further testing, with pharmokinetics.

**Example 7**

A study was conducted, as follows, to evaluate the exposure of Compound 1 following oral (PO) administration of three formulations of the compound in fasted male Cynomolgus monkeys. One of the formulations (Batch 21) was a suspension of 6% w/w Compound 1 in 0.5% w/w hydroxypropylmethyl cellulose (HPMC). The other two formulations tested were tablets from the wet granulation Batch 15 and from the dry granulation batch 20 prepared as described in Example 4, above.

The monkeys were separated into three groups of four animals each. Monkeys were fed in the afternoon prior to the day of dosing and the remaining food was removed at 7 pm. Food was returned at four hours post dosing. Each monkey received an oral dose of 30 mg of Compound 1 via rubber oral gavage tube or tablet (5 ml of 6 mg/ml of Compound 1 in suspension Batch 21, 2 tablets/animal of 15 mg per tablet of wet granulation Batch 15 or dry granulation Batch 20), and each dose was followed by a 3 ml flush using deionized water. Blood samples were drawn from each monkey at the following time points: pre-dose (0), 0.5, 1, 2, 3, 4, 6, 8, 12, 24, and 48 hours. Each sample was centrifuged for at a temperature of to 8oC at 3,000xg for 5 minutes, plasma was collected, and frozen on dry ice until testing. Plasma concentrations were determined by LC-MS/MS. Pharmacokinetics parameters were determined.

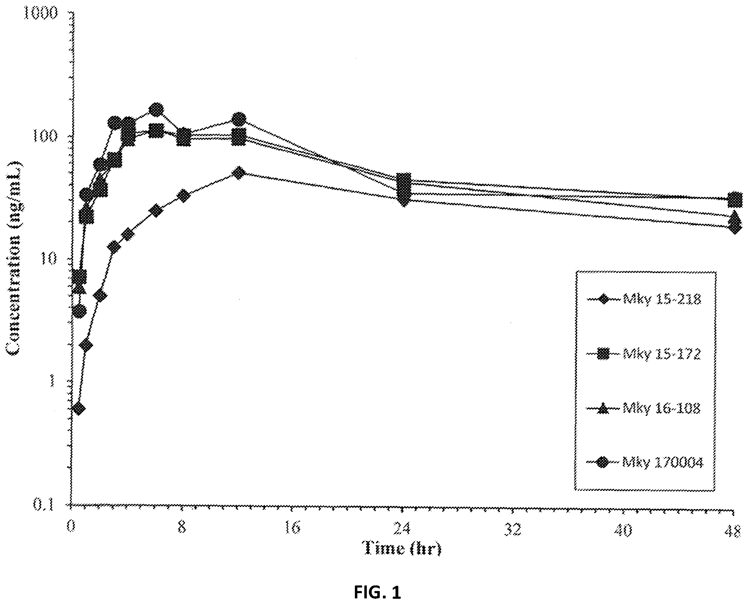

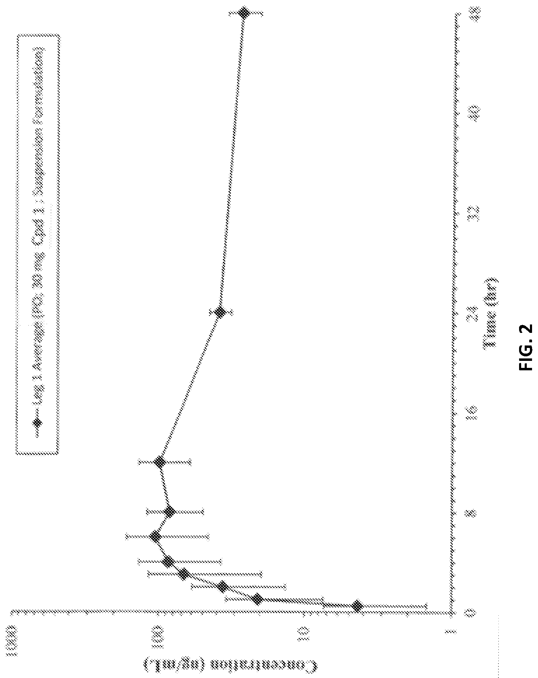

A plot of the individual plasma concentrations of Compound 1 after oral administration of the oral Compound 1 suspension formulation (Batch 21) in 0.5% HPMC in water at 30 mg in the male Cynomolgus Monkeys (Leg 1) is provided in FIG. 1. The four monkeys in the study are identified as "Mky 15-218," "Mky 15-172," "Mky 16-108," and "Mky 170004" in FIG. 1 and other figures below. A plot of mean plasma concentrations at each time point in Leg 1 is provided in FIG. 2. The results are summarized in Table 4, below:

**TABLE 4**

| Animal ID | 15-218 | 15-172 | 16-108 | 170004 | Mean (n=4) | SD |
|---|---|---|---|---|---|---|
| Animal Weight (kg) | 4.20 | 4.18 | 4.07 | 4.96 | 4.35 | 0.41 |
| Dosed (mg) | 30 | 30 | 30 | 30 | 30 | 0 |
| Dose (mg/kg) | 7.14 | 7.18 | 7.37 | 6.05 | 6.93 | 0.60 |
| $C_{max}$ (ng/mL) | 50.6 | 113 | 113 | 167 | 111 | 47.6 |
| $t_{max}$ (hr) | 12 | 6.0 | 6.0 | 6.0 | 7.5 | 3.0 |
| $t_{1/2}$ (hr) | ND | 22.6 | 18.7 | ND | 20.7 | ND |
| $AUC_{last}$ (hr·ng/mL) | 1388 | 2845 | 2623 | 3203 | 2515 | 788 |

As shown in Table 4, following PO dosing of the suspension formulation (Batch 21) at 30 mg/animal (Leg 1), maximum plasma concentrations (average of 111 ± 47.6 ng/mL) were observed between 6 and 12 hours post dosing. The average half-life following oral dosing was 20.7 hours. The average total exposure for Compound 1 (Leg 1) at 30 mg/animal was 2515 ± 788 hr*ng/mL and based on the dose normalized AUC last was 369 ± 138 hr*kg*ng/mL/mg.

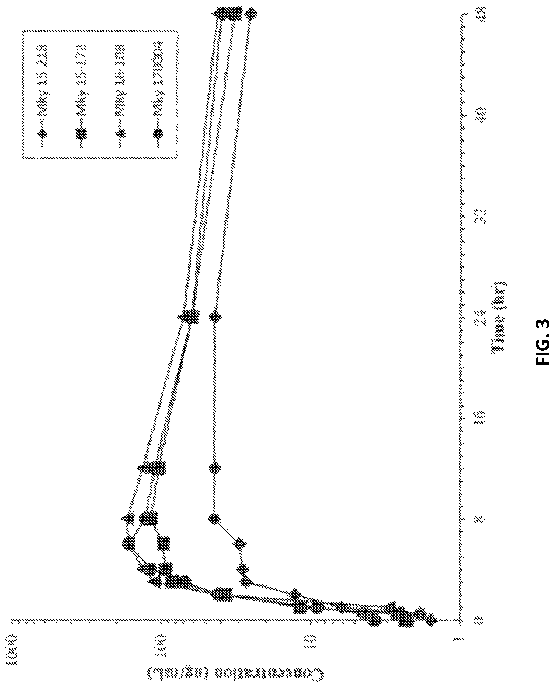

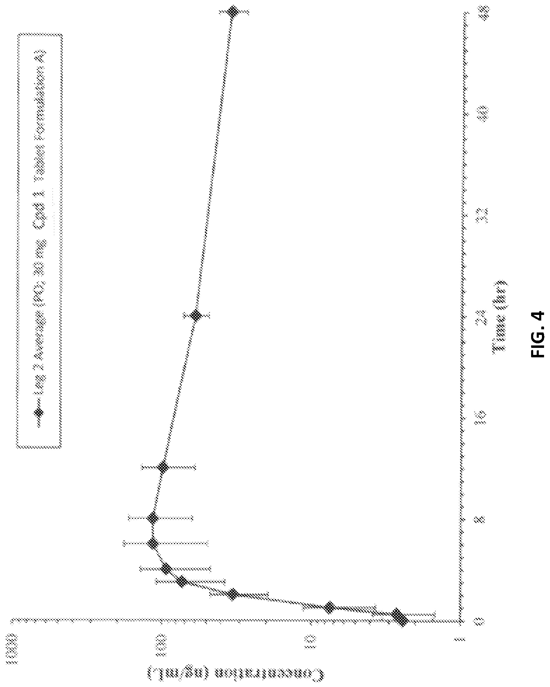

A plot of the individual plasma concentrations obtained from each monkey after oral administration of 30 mg/animal (Leg 2) of Tablet Formulation A (wet granulation Batch 15) is provided in FIG. 3. A plot of mean plasma concentration at each time point is provided in FIG. 4. The results of Leg 2 of the study are summarized in Table 5:

**TABLE 5**

| Animal ID | 15-218 | 15-172 | 16-108 | 170004 | Mean (n=4) | SD |
|---|---|---|---|---|---|---|
| Animal Weight (kg) | 4.21 | 4.36 | 4.00 | 4.95 | 4.38 | 0.41 |
| Dosed (mg) | 30 | 30 | 30 | 30 | 30 | 0 |
| Dose (mg/kg) | 7.13 | 6.88 | 7.50 | 6.06 | 6.89 | 0.61 |
| $C_{max}$ (ng/mL) | 43.7 | 118 | 170 | 166 | 124 | 58.8 |
| $t_{max}$ (hr) | 8.0 | 8.0 | 8.0 | 6.0 | 7.5 | 1.0 |
| $t_{1/2}$ (hr) | 43.4 | 22.0 | 22.8 | 23.7 | 28.0 | 10.3 |
| $AUC_{last}$ (hr·ng/mL) | 1718 | 1364 | 4052 | 3506 | 3110 | 997 |
| Relative F (%) | 124% | 107% | 157% | 110% | 124% | 23% |

As shown in Table 5, following PO dosing of tablet formulation A (wet granulation Batch 15) at 30 mg/animal (Leg 2), maximum plasma concentrations (average of 124 ± 58.8 ng/mL) were observed between 6 and 8 hours post dosing. The average half-life following oral dosing was 28.0 ± 10.3 hours. The average total exposure for Compound 1 (Leg 2) at 30 mg/animal was 3110 ± 997 hr*ng/mL and based on the dose normalized $AUC_{last}$ was 455 ± 151 hr*kg*ng/mL/mg.

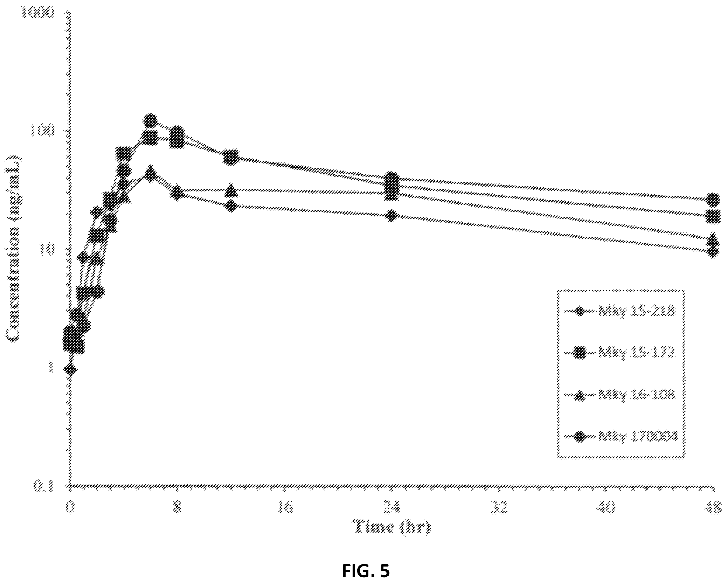

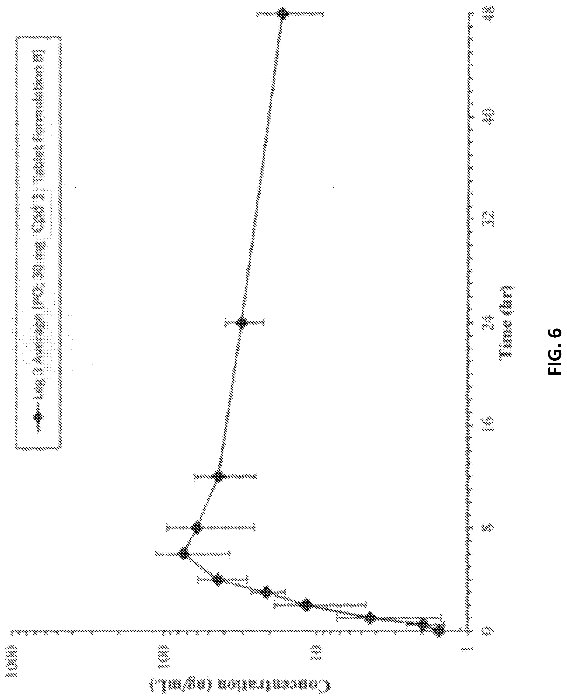

A plot of the individual plasma concentrations obtained from each monkey after oral administration of 30 mg/animal (Leg 3) of Tablet Formulation B (dry granulation Batch 20) is provided in FIG. 5. A plot of mean plasma concentration at each time point is provided in FIG. 6. Results of Leg 4 of the study are summarized in Table 6, where * indicates $p < 0.05$ when compared to $AUC_{last}$ from suspension formulation.

**TABLE 6**

| Animal ID | 15-218 | 15-172 | 16-108 | 170004 | Mean (n=4) | SD |
|---|---|---|---|---|---|---|
| Animal Weight (kg) | 4.49 | 4.46 | 4.32 | 5.14 | 4.60 | 0.37 |
| Dosed (mg) | 30 | 30 | 30 | 30 | 30 | 0 |
| Dose (mg/kg) | 6.68 | 6.73 | 6.94 | 5.84 | 6.55 | 0.49 |
| $C_{max}$ (ng/mL) | 41.5 | 87.1 | 45.5 | 120 | 73.5 | 37.2 |
| $t_{max}$ (hr) | 6.0 | 6.0 | 6.0 | 6.0 | 6.0 | 0.0 |
| $t_{1/2}$ (hr) | 26.0 | 22.4 | 24.8 | 32.2 | 26.4 | 4.20 |

(continued)

| Animal ID | 15-218 | 15-172 | 16-108 | 170004 | Mean (n=4) | SD |
|---|---|---|---|---|---|---|
| AUC$_{last}$ (hr·ng/mL) | 920 | 1883 | 1185 | 2107 | 1524 | 562 |
| Relative F (%) | 62% | 62% | 42% | 63% | 58% | 10% |

As one can see from Table 6, following PO dosing of tablet formulation B (dry granulation Batch 20) at 30 mg/animal (Leg 3), maximum plasma concentrations (average of 73.5 ± 37.2 ng/mL) were observed at 6 hours post dosing. The average half-life following oral dosing was 26.4 ± 4.20 hours. The average total exposure for Compound 1 (Leg 3) at 30 mg/animal was 1524 ± 562 hr*ng/mL and based on the dose normalized AUC$_{last}$ was 237 ± 102 hr*kg*ng/mL/mg.

Based on the average dose normalized AUC$_{last}$ values, Tablet formulation A (wet granulation Batch 15) had an exposure of 455 hr*kg*ng/mL/mg, which is 124 ± 23% of the exposure from suspension formulation (369 hr*kg*ng/mL/mg). Tablet formulation B (dry granulation Batch 20) had an exposure of 237 hr*kg*ng/mL/mg, which is 58 ± 10% of the exposure from suspension formulation. Thus, the AUC from solid formulation A was found to be very comparable to the one from the suspension formulation. However, the AUC from solid formulation B was significantly lower (P<0.05) compared to the value from the suspension formulation. In other words, these studies show that Compound 1 was significantly more bioavailable in the tablets produced by wet granulation (formulation Batch 15) than in the suspension formulation or in the tablets produced by dry granulation (formulation Batch 20).

Example 8

Using the tablets of wet granulation formula Batch 15 as a starting point, additional studies were conducted to identify excipients and concentrations of each excipient that could be scaled up and readily processed during the tableting process, and which had superior physical characteristics, including rapid dissolution characteristics. Grades of lactose and cellulose were selected that are particularly suitable for wet granulation processes and the total amount of intragranular excipients was increased. The concentration of Povidone was increased from 1% to 2% to 5% in three different batches. The total amount of lactose monohydrate used in the formulation was also increased, and the ratio of mcc to lactose monohydrate was reduced. Examples of three formulations prepared and tested are provided in Table 7, below. 500 gram batches of 50 g of Compound 1 per batch were prepared of each formulation below.

TABLE 7

| Batch No | 22 | 23 | 24 | 25 |
|---|---|---|---|---|
| Ingredient name | %w/w | %w/w | %w/w | %w/w |
| Intragranular | | | | |
| Compound 1 | 10.0 | 10.0 | 10.0 | 10.0 |
| Microcrystalline cellulose 101 | 25.0 | 20.0 | 20.0 | 20.0 |
| Lactose monohydrate 310 | 15.0 | 40.0 | 40.0 | 40.0 |
| Povidone K30 | 1.5 | 2.0 | 5.0 | 3.0 |
| Croscarmellose sodium | 2.5 | 2.5 | 2.5 | 2.5 |
| Poloxamer 407 | 1.0 | | | |
| Water USP | 30% IP | 25% IP | 40% IP | 40% IP |
| Subtotal (dry basis) | 55.0 | 74.5 | 77.5 | 77.5 |
| Extragranular | | | | |
| Lactose monohydrate 316 | 41.0 | 20.0 | 17.0 | 19.0 |
| Croscarmellose sodium | 2.5 | 2.5 | 2.5 | 2.5 |
| Poloxamer 407 | | 1.0 | 1.0 | 1.0 |
| Colloidal silicon dioxide | 0.5 | 0.5 | 0.5 | 0.5 |
| Magnesium stearate MF-3-V | 1.0 | 1.5 | 1.5 | 1.5 |

(continued)

| Batch No | 22 | 23 | 24 | 25 |
|---|---|---|---|---|
| Ingredient name | %w/w | %w/w | %w/w | %w/w |
| Total (dry basis) | 100.0 | 100,0 | 100.0 | 100.0 |

Tablets produced by wet granulation with the compositions described above were coated, but the coating did not affect the disintegration time. Table 8, below, shows the results from testing tablet cores produced from batches 23-25, above, some with 5 mg (A) and others with 50 mg (B) of Compound 1.

TABLE 8

| Cores (Strength/Tooling) | Weight (mg) | Thickness (mm) | Hardness (kp) | Friability (%) | Disintegration (min) |
|---|---|---|---|---|---|
| | n-10/Reported as Average (Minimum-Maximum) | | | wt: 6.5 g | N=6 |
| Batch 23A (5mg/5mm) | 52.1 (51.7-52.6) | 2.50 (2.47-2.53) | 3.5 (3.1-3.9) | 00.07 | First: 5.2 Last: 6.1 |
| Batch 23B (50mg/11mm) | 509 (507-510) | 5.08 (5.05-5.10) | 9.9 (8.9-10.6) | 0.17 | First: 3.5 Last: 4.0 |
| Batch 24B (50mg/11mm) | 508 (504-512) | 4.99 (4.95-5.02) | 9.9 (9.6-11.1) | 0.17 | First: 17.3 Last: 18.8 |
| Batch 25A (5mm/11mm) | 51.7 (50.7-52.6) | 2.54 (2.52-2.56) | 2.3 (2.0-2.5) | 0.08 | First: 6.0 Last: 6.8 |
| Batch 25B (50mg/11mm) | 510 (508-515) | 5.14 (5.12-5.17) | 10.6 (10.0-11.5) | 0.14 | First: 11.3 Last: 12.6 |

Sticking occurred in batch 22, with a PVP level of 1% and the formulation exhibited a large amount of fines when the formulation was scaled up to 500 g. Due to such issues, the compression of this lot was aborted.

Batch 23, with a PVP level of 2%, was found to compress into tablets with no sticking issues.

For Batch 24, with a PVP level of 5%, disintegration time of produced tablets increased considerably to 17 min. The final blend of Batch 24 also showed segregation between granules and powdered extra-granular excipients.

Batch 25, with PVP level of 3% showed a disintegration time between that of batches 23 and 24, indicative of the role of PVP as a binder.

**Example 9**

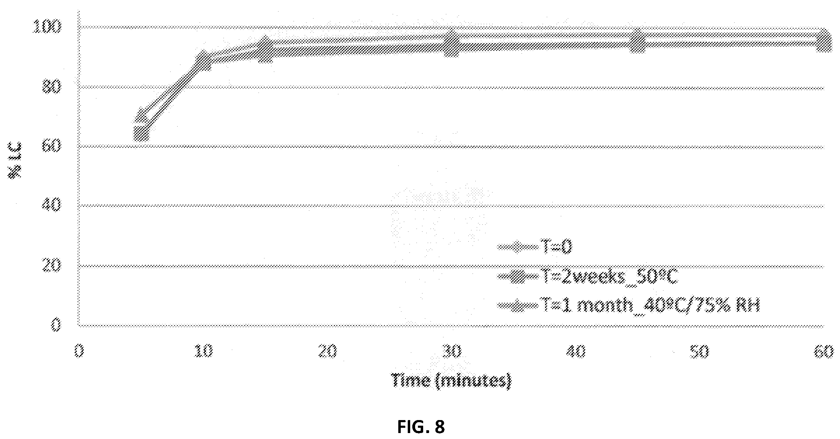

Tablets produced from Batch 23 (with 2% PVP) containing 5 mg and 50 mg of Compound 1, respectively, as described above were tested for stability after storage for 2 weeks at 50°C and after 1 month at 40°C/75% relative humidity. Dissolution was carried out in 500 ml of 0.01N HCl, Apparatus II, stirred at 75 revolutions per min. The tablets showed chemical stability by no increase in related substances observed. The resulting dissolution profiles are illustrated in Figures 8 (5 mg tablet) and 9 (50 mg tablet). The dissolution profiles show immediate release of Compound 1 from each tablet, and comparable profiles to initial, even after storage at the higher temperature and humidity.

**Example 10: Phase 1 Clinical Study Protocol**

Phase 1 Dose Escalation Study was initiated to assess the safety and pharmacokinetics of Compound 1 Oral Tablets (5 mg and 50 mg) compared to placebo in healthy subjects.

**Primary Study Objectives:**

(i) To characterize the safety and tolerability of single ascending doses of Compound 1 in healthy subjects; (ii) To characterize the safety and tolerability of Compound 1 administered for 14 or up to 21 days in healthy subjects; (iii) To characterize the pharmacokinetics in plasma and cerebrospinal fluid (CSF) after administration of Compound 1 for 7 days in healthy subjects; (iv) To characterize the food effect on the pharmacokinetics (PK) in plasma of Compound 1 after administration of a single dose of Compound 1 in healthy subjects; and, (v) To characterize the safety and tolerability of Compound 1 administered for up to 28 days in healthy subjects.

**Secondary Study Objectives:**

(i) To characterize the pharmacokinetics of single doses of Compound 1 in healthy subjects; (ii) To characterize the pharmacokinetics of Compound 1 administered for 14 or up to 21 days in healthy subjects; (iii) To assess the QTc and drug concentration effect of Compound 1 after repeated ascending doses; (iv) To assess safety and tolerability of Compound 1 after administration for 7 days in healthy subjects; (v) To characterize the safety and tolerability of single doses of Compound 1 administered in the fed state in healthy subjects; and, (vi) To characterize the pharmacokinetics of Compound 1 administered for up to 28 days in healthy subjects.

**Exploratory Study Objectives:**

(i) To explore the effect of single dose of Compound 1 administered on huntingtin (HTT) premRNA splicing in the blood of healthy subjects; (i) To explore the effect of Compound 1 administered for 14 or up to 21 days on HTT pre-mRNA splicing and HTT protein levels in the blood of healthy subjects; (ii) To explore the effect of single dose of Compound 1 administered (with food) on HTT pre-mRNA splicing in the blood of healthy subjects; and, (iii) To explore the effect of Compound 1 administered for up to 28 days on HTT premRNA splicing and HTT protein levels in the blood of healthy subjects.

**Study Design:**

The Phase 1 study was conducted in 5 parts: single ascending doses (SAD)(Part 1), multiple ascending doses (MAD)(Part 2), CSF and blood sampling after 7 days of Compound 1 administration (Part 3), food effect (Part 4), and multiple dosing for up to 28 days (Part 5). Part 1, Part 2, and Part 5 are doubleblind; Part 3 and Part 4 are open-label. Note that Part 3, Part 4, and Part 5 may be conducted concurrently.

**Study Methodology:**

The study was monitored by a Safety Review Committee (SRC). The intent of the SRC was to ensure that treatment does not pose undue risk to subjects. Safety and tolerability was assessed by the SRC between each cohort prior to ascending from one dose level to the nexthigher dose level in Part 1 (single ascending dose [SAD]) and Part 2 (multiple ascending dose [MAD]), and prior to initiating Part 3 (CSF), Part 4 (FE), and Part 5.

The SRC was composed of the following personnel: Principal Investigator or delegate (delegation only when the Principal Investigator is not available); Sponsor medical monitor or delegate (must be a physician); Other internal or external experts may be invited to participate in the review or may be consulted.

The parts of the study were not necessarily be conducted in numerical sequence and may run concurrently. The SRC met prior to the initiation of Part 5 to determine the doses to be used in this portion of the study. Doses (which may include loading and maintenance doses) were selected prior to initiation of Part 5 based on the available SAD and MAD data. The SRC did not plan to meet between cohorts within Part 5.

**Part 1 (SAD):**

The single ascending dose (SAD) part of the study was randomized, double-blinded, and placebo controlled in healthy male and female subjects.

Five dose levels are planned to be tested in 5 cohorts of 8 subjects each (Cohort 1.1 to 1.5). However, the Sponsor may elect to evaluate an additional cohort(s).

The initial dose in the first cohort was ≤1/10 of the human equivalent dose (HED) estimated from the NOAEL (no observed adverse effect level) of the (male) rat, which is the most sensitive species, following the FDA guidance on the maximum recommended starting dose (MRSD) and EMA guidelines. The NOAEL of the rat is 6 mg/kg. This was set by the observation in male rats of germ cell exfoliation in epididymis and testes. The HED of 0.97 mg/kg was calculated; this scaled in a 70 kg human to 68 mg. Adjusting this dose to 1/10, the dose of the first cohort was 6.8; the actual administered dose will be 5 mg.

In Cohort 1.1, sentinel dosing was performed in 2 subjects (1 subject with Compound 1 and 1 subject with placebo). The remaining subjects in this cohort were dosed at least 24 hours later, if no clinically significant safety issues are observed. The remaining 6 subjects (5 subjects with Compound 1 and 1 subject with placebo) may be dosed as a group. Cohort 1.1 was the only cohort in which sentinel dosing was performed. In subsequent cohorts, all 8 subjects may be dosed as a group.

After each cohort completed dosing, a dose escalation meeting was to take place. The dose level for the next cohort would be based upon the PK and safety from the previous cohort. The incremental increase in dose was determined by the relationship of mean exposure in the cohort to that of the NOAEL.

If the mean area under the curve (AUC) was <1/10 of that at the NOAEL, the dose may be increased by up to 200%. That is, the subsequent dose may be up to three times the prior dose.

If the mean AUC was between $\geq$1/10 and <1/5 of the AUC at the NOAEL, the dose may be increased by up to 100%. That is, the subsequent dose may be up to two times the prior dose.

If the mean AUC was between $\geq$1/5 and <1/2 of the AUC at the NOAEL, the dose may be increased by up to 50%. That is, the subsequent dose may be up to one- and one-half times the prior dose.

The highest dose level was that associated with a mean exposure not exceeding 1/2 of the AUC at the NOAEL; no additional escalations were to be performed. The dose escalation was to continue unless dose escalation stopping criteria were met.

Eligibility was to be assessed during a screening period of up to 28 days. Subjects were to into the clinic 1 day before dosing (Day -1). On the morning of Day 1, Compound 1 or placebo were orally administered after an overnight fast of at least 10 hours. Subjects were released from the clinic on Day 8 after all required study procedures are completed and if medically appropriate. A follow-up safety phone call was to occur 4 weeks ($\pm$1 week) after discharge on Day 8.

**Part 2 (MAD):**

The multiple ascending (MAD) part of the study was randomized, double-blind, and placebo controlled in healthy male and female subjects. Up to five regimens are planned to be tested in up to 5 cohorts of 8 subjects each (Cohort 2.1 to 2.5). Within each cohort, 6 subjects were to receive Compound 1 and 2 subjects were to receive placebo. Subjects in Cohort 2.1 and 2.2 were to be dosed for 14 days, subjects in Cohort 2.3 to 2.5 were to be dosed for up to 21 days.

Part 2 may be initiated once at least 2 cohorts in Part 1 have been dosed, safety parameters have been reviewed, the respective SAD PK parameters have been calculated, and MAD dosing simulations of corresponding SAD doses have been performed. Selection of specific multiple dose levels were to be informed by available SAD PK data, simulations and general safety observed in Part 1. After the dose levels have been evaluated in once-daily format, a pharmacokinetic simulation was to be performed to determine the fluctuation within the dosing interval. In Cohort 2.3, dosing on Day 1 and 2 were to be with a loading dose that was to be higher than the dose selected for the remainder of the scheduled doses. A similar dosing schedule may be selected for Cohort 2.4 and 2.5. Alternative dosing schedules may be considered for all cohorts in Part 2 if data collected and analyzed during the study warrant it.

Eligibility was to be assessed during a screening period of up to 28 days. Subjects were to check into the clinic 1 day before dosing (Day -1). On each morning of the scheduled dosing period (ie, Day 1 up to Day 21), Compound 1 or placebo were to be orally administered after an overnight fast of at least 10 hours. Subjects were to be released from the clinic 7 days after the last dose (ie, Day 21 or up to Day 28) and after all required study procedures are completed and if medically appropriate. Subjects were to return to the clinic for an ambulant visit 7 days after release (ie, Day 28 or up to Day 35) for the collection of PK and PD (mRNA and HTT protein) samples. A follow-up safety phone call or ambulant visit was to occur on Day 49 ($\pm$7 days).

**Part 3 (CSF):**

The concentrations of Compound 1 in plasma and CSF were to be assessed in an open-label design in healthy male and female subjects. A single dose of Compound 1 was to be administered daily for 7 days in 1 cohort of 6 subjects (Cohort 3.1). The dose level of Part 3 was to be determined based upon a review of the safety, tolerability, and PK data of Part 1 and Part 2 of the study. While the MAD dose was to be determined further in development, that dose and schedule was to be applied to this part of the study.

Eligibility was to be assessed during a screening period of up to 28 days. Subjects were to check into the clinic 1 day before dosing (Day -1). On the morning of Day 1 to Day 7, Compound 1 was to be orally administered after an overnight fast of at least 10 hours each day. Serial sampling of CSF and sampling of plasma for drug concentrations was to be performed on Day 7. The exact timing of the CSF and blood samples was to be determined based on the results of Part 1 and Part 2. Subjects were to be released from the clinic on Day 9 after all required study procedures are completed and if medically appropriate. A follow-up safety phone call was to occur 4 weeks ($\pm$1 week) after discharge on Day 9.

**Part 4 (FE):**

The food effect (FE) part was a parallel, open-label part in healthy male and female subjects in up to 3 cohorts of 6 subjects each. Up to 3 dose levels of Compound 1 was to be administered 30 minutes after the start of a high-fat, high calorie breakfast. Part 4 may be initiated when sufficient data of Part 1 are available. The dose levels for this part were to be chosen based upon a review of available safety, tolerability and PK data as determined in Part 1 and Part 2.

Eligibility was to be assessed during a screening period of up to 28 days. Subjects were to check into the clinic 1 day before dosing (Day -1). On the morning of Day 1, Compound 1 was to be orally administered after ingestion of a standardized, high-fat, high calorie breakfast. Subjects are released from the clinic on Day 8 after all required study procedures are completed and if medically appropriate. A follow-up safety phone call was to occur 4 weeks ($\pm$1 week) after discharge on Day 8.

**Part 5 (Multiple Dosing for up to 28 days [MD28D]):**

Part 5 was a randomized, double-blind, and placebo-controlled assessment of multiple doses for up to 28 days in healthy male and female subjects. Up to 3 cohorts of 8 subjects each are planned. Prior to the initiation of Part 5, the SRC was to meet for selection of dose (which may include loading and maintenance doses), dosing regimen (including fed or fasted condition), and duration (up to 28 days) for this part of the study based upon available data from the completed cohorts of Part 1 and Part 2. Within each cohort, 6 subjects were to receive Compound 1, and 2 subjects were to receive placebo. The total dose on any day was not to exceed doses that were established as well tolerated in Part 1 (SAD).

Eligibility was to be assessed during a screening period of up to 28 days. Subjects were to check into the clinic 1 day before dosing (Day -1). On each day of dosing, Compound 1 or placebo was to be orally administered in the morning either after an overnight fast or following a standard highfat meal, per the SRC determined regimen selected for a given cohort. Subjects were to be released from the clinic 7 days after the final dose and after all required study procedures are completed and if medically appropriate. Subjects were to return to the clinic for an ambulant visit 7 days after being released from the clinic for the collection of PK and PD (mRNA and HTT protein) samples, and safety assessments. On Day 1 and on the day of anticipated maximum exposure (ie, either Day 2 or, if loading doses are not used, Day 29) patients were to be monitored with a 24-hour Holter monitor device.

**Study Population:**

**Part 1:** Up to 48 male and female subjects between 18 and 65 years of age, inclusive.

**Part 2:** Up to 40 male and female subjects between 18 and 65 years of age, inclusive.

**Part 3:** 6 male and female subjects between 50 and 65 years of age, inclusive.

**Part 4:** Up to 18 male and female subjects between 18 and 65 years of age, inclusive.

**Part 5:** Up to 24 male and female subjects between 18 and 65 years of age, inclusive.

**Inclusion Criteria:**

The following criteria must be met by all subjects to be considered for study participation:
For Part 1, Part 2, Part 4, and Part 5: Healthy male or female subjects aged from 18 to 65 years old, inclusive, at Screening.
For Part 3: healthy male of female subjects aged 50 to 65 years old, inclusive, at Screening.

Subjects must understand the nature of the study and must provide signed and dated written informed consent before the conduct of any study-related procedures.

Body Mass Index (BMI) of $\geq$18.5 kg/m$^2$ and $\leq$30.0 kg/m$^2$ with a body weight $\geq$50.0 kg for male subjects and a body weight $\geq$45.0 kg for female subjects at Screening.

Healthy as determined by the Investigator, based upon a medical evaluation including medical history, physical examination, laboratory test results, ECG recording (eg, QTcF $\leq$ 450 msec for males and QTcF $\leq$ 470 ms for females) and vital signs. Out of range values can be repeated once.

Male subjects and female subjects of childbearing potential must be willing to use 2 methods of birth control for the duration of the study and for 30 days after the last dosing.

Postmenopausal female subjects must have had $\geq$12 months of spontaneous amenorrhea (with follicle-stimulating hormone (FSH) $\geq$30 mIU/mL at Screening). Surgically sterile women are defined as those who have had a hysterectomy, bilateral ovariectomy, or bilateral tubal ligation $\geq$6 months prior to Screening.

All female subjects of childbearing potential must have a negative serum pregnancy test result at Screening and a negative urine pregnancy test on Day -1.

Male subjects must agree to not donate sperm for the duration of the study and for at least 3 months after the last dosing.

Part 3 only: Subject must be willing to undergo lumbar puncture for CSF sampling.

Part 4 only: Subject must be willing and able to consume the entire high-fat breakfast in the designated timeframe.

**Exclusion Criteria:**

Subjects will be excluded when they meet any of the following criteria:

Subjects that participated in any drug or device clinical investigation within 60 days prior to Screening or who anticipate participating in any drug or device clinical investigation within the duration of this study.

Prior or ongoing medical condition (eg, concomitant illness, psychiatric condition), medical history, physical findings that, in the Investigator's opinion, could adversely affect the safety of the subject or could impair the assessment of study results.

An abnormal general neurological examination.

Presence of any clinically significant abnormality during Screening.

Any psychological, emotional problems, any disorders or resultant therapy that are likely to invalidate informed consent or limit the ability of the subject to comply with the protocol requirements.

A positive Hepatitis B surface antigen, positive Hepatitis C antibody or human immunodeficiency virus (HIV) antibody result at Screening.

Donation of plasma within 7 days prior to dosing. Donation or loss of blood (excluding volume drawn at screening or menses) of 50 mL to 499 mL of blood within 30 days, or more than 499 mL within 56 days prior to the dosing.

Excessive alcohol consumption (regular alcohol intake $\geq 21$ units per week for male subjects and $\geq 14$ units per week for female subjects) within 6 months prior to Screening. One unit (8 g) is equivalent to a ½ pint (280 mL) of beer, 1 measure (25 mL) of spirits or 1 small glass (125 mL) of wine.

The subject is a smoker or uses other nicotine-containing products. Ex-smokers must have ceased smoking >3 months prior to Screening.

A positive urine drug screen, cotinine screen or alcohol breath test at Screening or on Day 1 of each treatment period.

Females who are pregnant or nursing.

Subject has previously received Compound 1.

Part 3 only: Contraindication to lumbar puncture, eg, low platelet count, abnormal prothrombin time international normalized ratio (PT-INR), spinal deformities or other spinal conditions that in the judgment of the Investigator would preclude a lumbar puncture.

**Duration Of Treatment:**

Part 1: 1 day; Part 2: 14 days (Cohort 2.1 and 2.2) or up to 21 days (Cohort 2.3 to 2.5); Part 3: 7 days; **Part 4:** 1 day; **Part 5:** up to 28 days

**Criteria For Evaluation:**

**Efficacy:**

The following PK parameters were assessed wherever feasible on Part Day 1 (Single Dose) PK [Part 1 (SAD), Part 2 (MAD, Day 1), Part 4 (FE), and Part 5 (MD28D, Day 1)]: $C_{max}$; the maximum observed plasma concentration, $C_{max}/D$; Dose normalized $C_{max}$ (Part 1 only); $T_{max}$; the time to reach $C_{max}$; $AUC_{0-24}$ (Area under the concentration-time curve from 0 to 24 hours); $AUC_{0-72}$ (Area under the concentration-time curve from 0 to 72 hours); $AUC_{0-tau}$ (Area under the concentration-time curve within dosing interval, calculated by linear up/log down trapezoidal method, for Part 2 only); $AUC_{0-t}$ (Area under the concentration-time curve from time zero to time t, where t is the time of the last measured (or measurable) concentration ($C_t$), calculated by linear up/log down trapezoidal method (Parts 1 and 4 only); $AUC_{0-t}/D$ (Dose normalized AUC from time zero to the last quantifiable concentration, Part 1 only); $AUC_{0-inf}$; (Area under the concentration-time curve from time zero to infinity, $AUC_{0-inf} = AUC_{0-t} + C_t/\lambda_z$, where $\lambda_z$ is the terminal elimination rate constant, calculated by linear up/log down trapezoidal method (Parts 1 and 4 only); $AUC_{0-inf}/D$ (Dose normalized AUC from time zero to infinity, Part 1 only); $\lambda_z$ (Apparent terminal rate constant calculated by linear regression of the terminal linear portion of the log concentration vs. time curve, Parts 1 and 4 only); $t_{1/2}$ (Apparent terminal half-life calculated as $\ln(2)/\lambda_z$, Parts 1 and 4 only); CL/F (Total body clearance, calculated as Dose/$AUC_{0-inf}$, Parts 1 and 4 only); and, $V_z/F$ (Apparent volume of distribution, calculated as Dose/($\lambda_z * AUC_{0-int}$)).

The following PK parameters were assessed wherever feasible on Day 14, Day 21, or Day 28 (Multiple Dose) PK [Part 2 (MAD) Cohort 2.1 and 2.2 (Day 14), Cohort 2.3 to 2.5 (Day 21), and Part 5 (MAD) Cohort 5.1 to 5.3 (Day 28)]: $C_{max}$ (The maximum observed plasma concentration over a dosing interval); $T_{max}$ (The time to reach $C_{max}$ over a dosing interval); $C_{min}$ (The minimum concentration over a dosing interval); $C_{avg}$ (Average concentration over a dosing interval); $AUC_{0-tau}$ (Area under the concentration-time curve within dosing interval, calculated by linear up/log down trapezoidal method); $AUC_{0-tau}/D$ (Dose normalized $AUC_{0-tau}$); $\lambda_z$ (Apparent terminal rate constant calculated by linear regression of the terminal linear portion of the log concentration vs. time curve); $t_{1/2}$ (Apparent terminal half-life calculated as $\ln(2)/\lambda_z$); CL/F (Total body clearance, calculated as Dose/$AUC_{0-tau}$); $V_z$/F (Apparent volume of distribution, calculated as Dose/($\lambda_z$*$AUC_{0-tau}$)); $AUCR_{auc}$ (Accumulation ratio based on $AUC_{0-tau}$: $AUC_{0-tau}$ on Last Dose*/$AUC_{0-tau}$ on Day 1); and, $AUCR_{cmax}$ (Accumulation ratio based on $C_{max}$: $C_{max}$ on Last Dose*/$C_{max}$ on Day 1).

The following PK parameters were assessed wherever feasible for *Last Dose on Day 14 for Part 2 (Cohort 2.1 and 2.2) or Day 21 for Part 2 (Cohort 2.3 to 2.5) or Day 28 for Part 5 (Cohort 5.1 to 5.3) and Day 7 (Multiple Dose) PK [Part 3 (Day 7)]:

$C_{max}$ (The maximum observed plasma concentration); $T_{max}$ (The time to reach $C_{max}$); $AUC_{0.5-12}$ (Area under the concentration-time curve from time 0.5 to 12 hours , calculated by linear up/log down trapezoidal method); and, CSF/Plasma ratio (Concentration ratios in CSF over plasma (Part 3 only)).

## Safety:

The following parameters were defined as parameters regarding safety and tolerability:

Change from baseline to each scheduled time point up to EOS for vital signs; Change from baseline to each scheduled time point up to EOS for ECG parameters; Change from baseline to each scheduled time point up to EOS for clinical laboratory tests; Changes from baseline in C-SSRS scores (Part 2, Part 3, and Part 5 only); Treatment-emergent adverse events (AEs) up to EOS; Treatment-emergent AEs leading to premature discontinuation of study drug; Treatment-emergent serious adverse events (SAEs) up to EOS; and, Abnormalities in physical examination.

## Statistical Methods:

## Pharmacokinetics:

Individual subject listings were provided. Mean and individual plasma concentration-time profiles for Compound 1 were presented graphically for each group.

PK variables were to be summarized using arithmetic mean, standard deviation, geometric mean, median, minimum, maximum, and CV%.

Attainment of steady state conditions were to be determined by visual inspection of the trough plasma concentrations.

To assess the effect of food, the PK parameters of Compound 1 in fasted (Part 1) and fed (Part 4) condition were to be graphically displayed, and descriptive statistics were to be prepared. Statistical analysis per dose level were to be performed in 6 subjects for Compound 1 using treatment in fed condition as test (Part 4) and the treatment with the same dose in fasted condition as reference (Part 1).

The primary PK parameters were to be $C_{max}$, $AUC_{0-t}$, and $AUC_{0-inf}$. The PK parameters of $C_{max}$, $AUC_{0-t}$, and $AUC_{0-inf}$ were to be naturally log-transformed first, and the means of these logtransformed parameters were to be estimated by the linear model with treatment (Compound 1 administered under fed conditions over that of Compound 1 administered under fasting conditions) as the only fixed factor. The difference of these means (in log scale) and its 90% confidence interval (CI) were to be exponentiated to form the ratio of geometric means (GMR) and corresponding CI for the ratio. Absence of food effect were to be concluded if all 90% CI results of the GMRs for the $C_{max}$, $AUC_{0-t}$, and $AUC_{0-inf}$ are contained within the interval 80.00% - 125.00%.

## Safety:

All safety parameters were to be summarized by dose level in Part 1 through Part 5.

Summary statistics (mean, median, standard deviation, minimum, maximum, and number of available observations) were to be provided for continuous demographic variables (eg, age, height, and weight). Individual subject listings of demographic data were to be provided.

Qualitative demographic characteristics (gender, race) were to be summarized by counts and percentages. Other baseline subject characteristics (eg, medical history, physical examination clinical findings, previous medications, and inclusion/exclusion checklist) were to only be listed.

ECG variables, vital sign measurements and laboratory measurements were to be summarized at each time point using mean, median, standard deviation, min, max, number of available observations, and change from baseline. C-SSRS parameters were to be analyzed using descriptive statistics where appropriate. Individual subject listings of ECG data, vital signs data, laboratory measurements and C-SSRS (Part 2, Part 3, and Part 5 only) were to be provided.

Distributions of these parameters were to be compared between the treatment groups (fasted or fed) only descriptively. No statistical inference were to be performed.

Holter analysis/Compound 1 plasma concentration-QTc effects may be performed, and results were to be provided in a separate report.

**Phase 1 Study Results**

The key objectives of the Phase 1 healthy volunteer trial were to establish a target dose range of Compound 1 Compound for lowering HTT mRNA and protein. The trial consisted of single (SAD) ascending dose (SAD) and multiple (MAD) ascending dose (MAD) cohorts. The dosing in all cohorts was well-tolerated with no safety-related findings, exhibiting dose-dependent splicing of HTT mRNA. The study duration for the MAD cohort was of a longer duration, enabling longer-term evaluation of *HTT* mRNA splicing and HTT protein lowering. The MAD cohort demonstrated that Compound 1 showed a long drug half-life, with maintenance of splicing up to 72 hours following the last dose.

The CSF sampling enabled the evaluation of pharmacokinetics of Compound 1 in the CSF wherein Compound 1 levels in the CSF were compared with Compound 1 levels in plasma. The Phase 1 Study results demonstrated that Compound 1 levels in the CSF were equal to or greater than levels observed in plasma. The food effect portion enabled the evaluation of pharmacokinetics of Compound 1 in plasma after administration of a single dose of Compound 1 in healthy subjects.

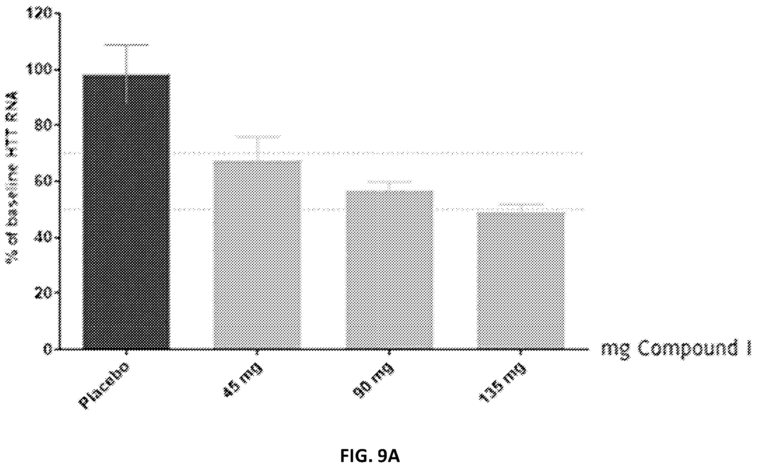

As shown in Figure 9A, the SAD cohort resulted in a dose-dependent lowering of HTT mRNA in whole blood taken from healthy volunteers 24 hours after they were administered with either placebo, 5 mg, 15 mg, 45 mg, 90 mg, or 135 mg of Compound 1.

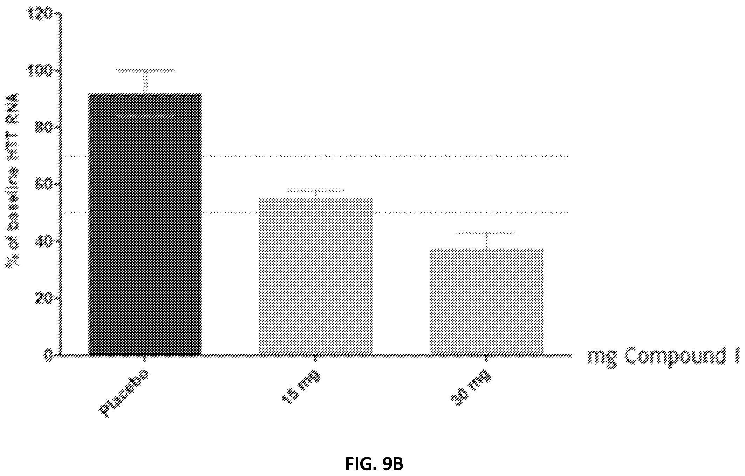

Similarly, the MAD cohort (Figure 9B) also showed a dose-dependent lowering of HTT mRNA in whole blood taken from healthy volunteers dosed with either placebo, 15 mg or 30 mg of Compound 1 for 14 days. The amount of HTT mRNA was then evaluated by RT-PCR 6 hours after administration of Compound 1 on day 14.

The target level of 30-50% lowering was achieved with the lowest dose tested both in the SAD and MAD cohorts. The half-life of HTT mRNA was estimated to be about 24 hours. Thus, after one day, if no HTT mRNA was synthesized, the total amount of HTT mRNA would be predicted to be about 50% of baseline. The administration of Compound 1 Compound in the SAD cohort essentially inhibited all *de novo* HTT mRNA synthesis. Thus, even with higher concentrations of Compound 1, the total amount of HTT mRNA remained at about 50% of baseline representing the amount of HTT mRNA synthesized prior to the administration of Compound 1.

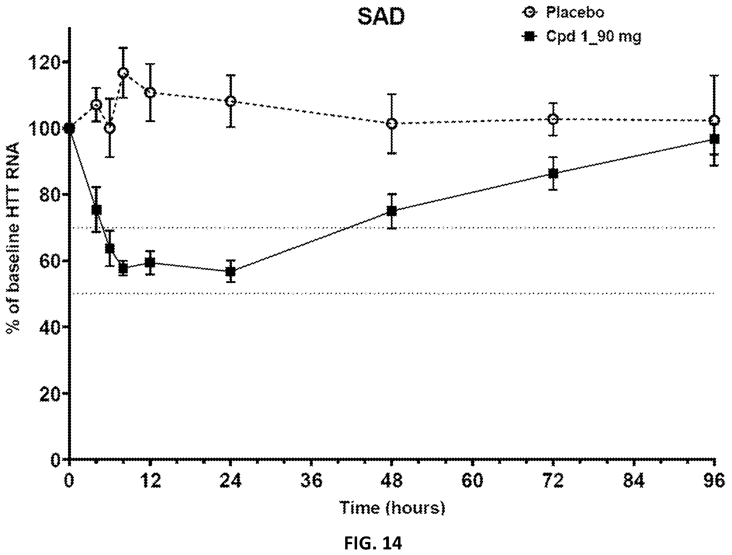

Results from measurement of HTT mRNA in the whole blood of subjects in the SAD cohorts are illustrated in Figure 14. The results also show that the *HTT* splicing effect of Compound 1 is reversible and persists for 72 hours post cessation of treatment.

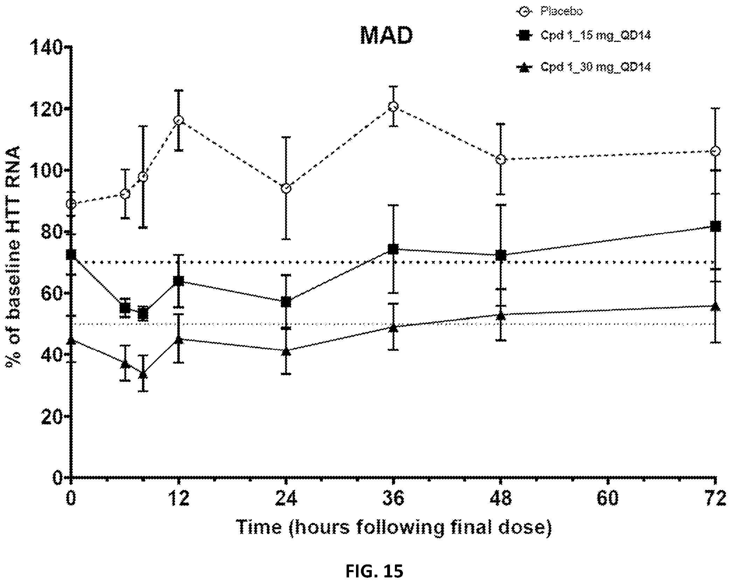

Results from measurement of HTT RNA in the whole blood of human subject administered a placebo or 15 or 30 mg of Compound 1, as described in the Multiple Ascending Dose (MAD) study above are illustrated in Figure 15. HTT splicing was monitored after the final dose at day 14, calculated as % HTT remaining from baseline (pre-dose day 0).

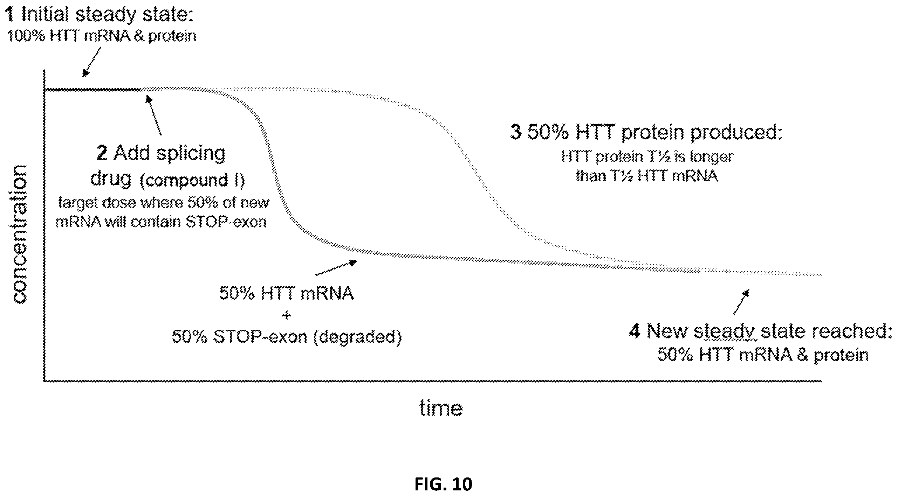

Figure 10 is an exemplary depiction of HTT mRNA and protein degradation kinetics that leads to a steady-state levels of RNA and protein.

In untreated cells, there is a steady state level of mRNA and protein because the amount of mRNA or protein being synthesized matches the amount that is being degraded, so that the mRNA and protein levels are the same over time. The addition of Compound 1 triggers the inclusion of the HTT pseudoexon into the transcript which results in the rapid decay of the HTT mRNA and a reduction of HTT mRNA to ~50% of baseline. The half-life of the HTT mRNA is about 24 hours. Hence, a day after drug treatment, the amount of HTT mRNA present is regulated by the dose of Compound 1. In this example, ~50% of newly synthesized mRNA was inhibited. Of the HTT mRNA synthesized prior to treatment, about 50% is degraded after 24 hours. The HTT protein level depends on how much mRNA is produced. Thus, a reduction by 50% would cause a 50% reduction of the HTT protein. However, HTT protein has a half-life of about 5-7 days, so it takes longer to get to the new steady state level. Finally, a new steady state is reached where 50% of the mRNA is present, and the new level of protein has fallen to 50% of the original amount. Changes in HTT protein levels were assessed in MAD cohort over a longer period of time. Accordingly, healthy subjects were treated for 21 days before the amount of HTT mRNA and protein was measured in blood samples taken from each subject.

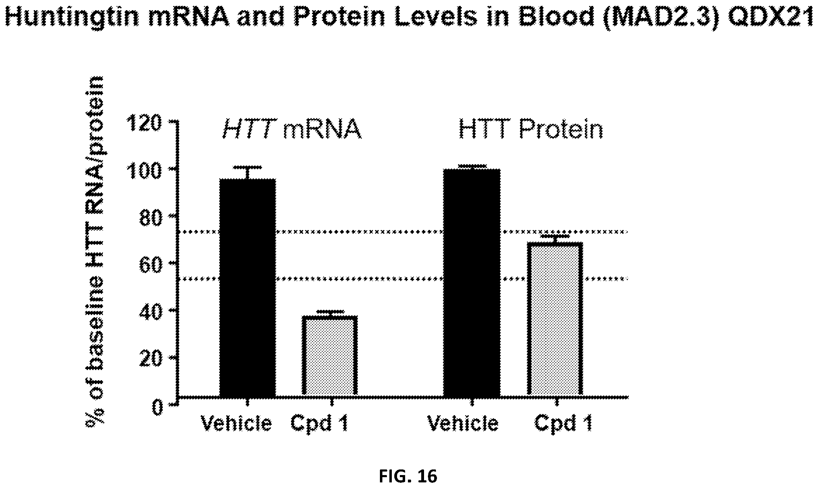

Figure 16 shows the huntingtin mRNA and protein levels measured in whole blood from MAD cohort 2.3 (30 mg administered for 21 days with 100 mg LD for 2 days), as described above, as a percent of baseline, after administration of vehicle or compound 1 to a human, 24 hours after the last dose. The results show *HTT* mRNA reduction reached steady state. Longer dosing was required for HTT protein levels to reach maximal steady state reduction. It is anticipated that the observed *HTT* mRNA changes in blood will result in similar decreases in HTT protein levels in Huntington's disease patients when steady state decrease in HTT is attained over time with continued treatment with Compound 1.

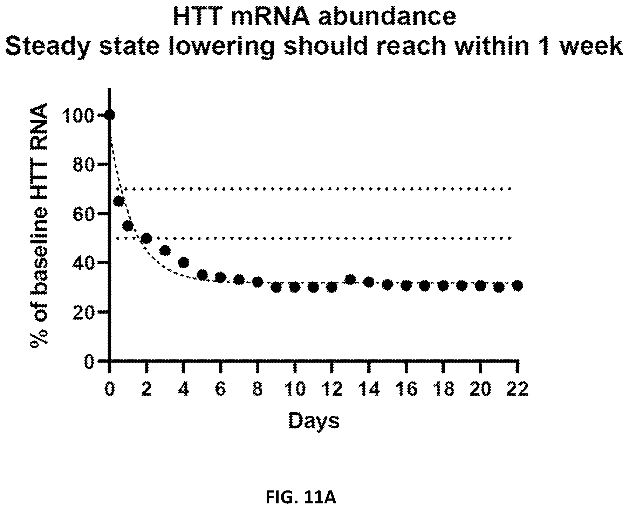

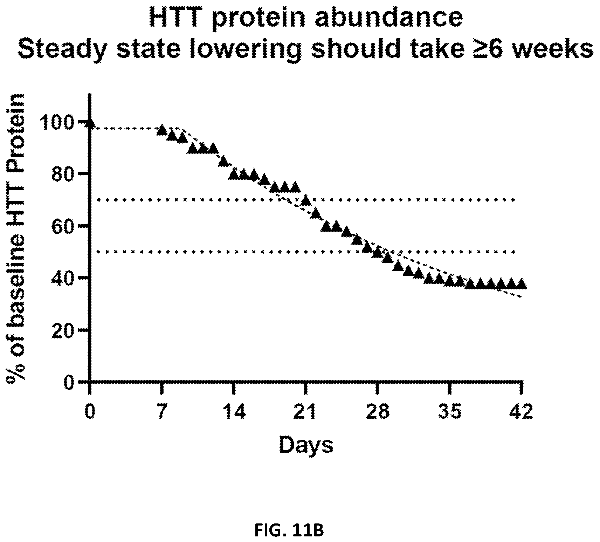

Figure 11 shows graphs that model the rate of HTT mRNA (Figure 11A) and HTT protein (Figure 11B) decay based on their half-lives and predict the time to reach steady state after Compound 1 treatment at 30 mg daily dose. For HTT mRNA, the half-life was estimated to be about 24 hours. HTT mRNA reaches steady state after approximately 5 days. For HTT protein, the half-life was estimated to be 5-7 days and consequently HTT protein steady state levels would only be attained about 6 weeks from the beginning of treatment.

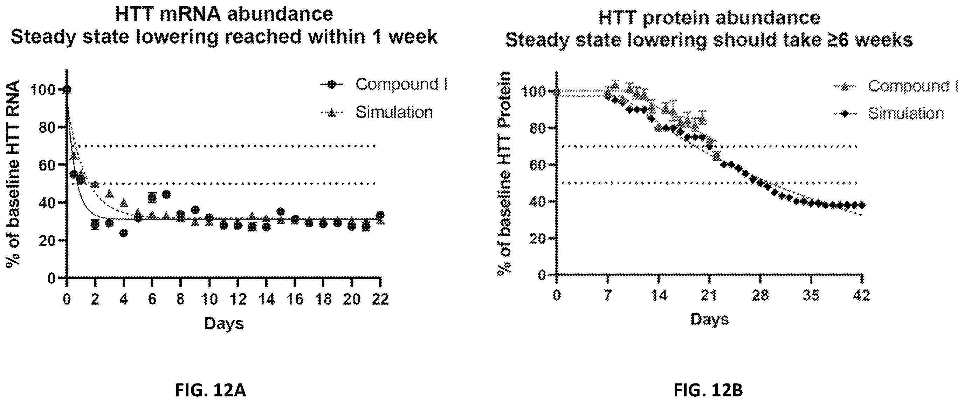

Figure 12 compares the trajectory of HTT mRNA (Figure 12A) and protein (Figure 12B) lowering seen in the Multiple Ascending Dose Study with those values predicted from the half-life of HTT mRNA and protein as shown in Figure 11. The results show that HTT mRNA levels rapidly decreased and reached steady state at about 4-5 days of treatment. As predicted, the rate of protein lowering was much slower, but after 21 days of treatment there was approximately 40% lowering in the amount of HTT protein. Equivalent steady state levels of HTT mRNA and protein could therefore be reached after about 4-5 weeks from the onset of treatment.

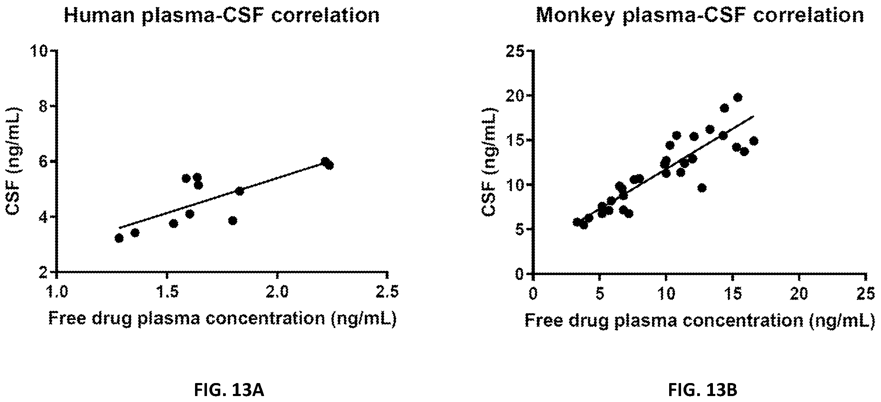

As shown in Figure 13, the level of Compound 1 in the cerebrospinal fluid (CSF) demonstrated that Compound 1 therefore crossed the blood brain barrier and was in direct correlation with the level of Compound 1 in free plasma both in humans (Figure 13A) and non-human primates (Figure 13B). The two subjects in this cohort received 30 mg daily dose. Compound 1 therefore crossed the blood brain barrier. The levels of Compound 1 found in the CSF were at least equivalent or greater than levels observed in the plasma, thus demonstrating in humans that Compound 1 was in humans, Compound 1 is not subject to efflux.

In the food effect cohort, Compound 1 showed similar exposures regardless of whether the subjects were fasted or fed.

In conclusion, the Phase I study demonstrated Compound 1 penetrated the blood brain barrier and selectively reduced HTT mRNA and protein in both the CNS and periphery in a dose dependent manner. These results confirm that exposure to Compound 1 in human patients leads to demonstrable reduction for both HTT mRNA and HTT protein.

**Example 11: Phase 2 Clinical Study Protocol**

A 12 week Phase 2, Randomized, Placebo-Controlled, Dose-Finding Study to Evaluate the Safety and Efficacy of Compound 1 in Subjects with Huntington's Disease.

Prior to the development of this Phase 2 study, Compound 1 was extensively evaluated in in vivo and in vitro preclinical pharmacology models, in a comprehensive toxicology program, and in an ongoing Phase 1 study in healthy volunteers. Together, the resulting data validate that Compound 1 treatment results in dose-dependent pre-mRNA splicing and reduced protein transcription and that Compound 1 treatment is safe and well tolerated in the clinic at single doses as high as 135 mg and multiple doses as high as 30 mg for 21 days.

The present 12-week double-blind study will allow for the quantification of the effect of Compound 1 on total HTT (tHTT) protein reduction in subjects with HD and evaluation of the safety of two doses over 12 weeks of Compound 1 treatment.

A parallel-group design was selected because it allows recruitment of patients for all treatment arms in the same timeframe. The time course in untreated patients for HTT protein, mRNA, and other indicators of drug response in the blood are not available. The use of a parallel arm design with concurrent placebo control allows a direct assessment comparison to determine the effect of active treatment.

The patient population was selected to reduce variability in an otherwise heterogeneous disease population by identifying subjects with active disease who have not yet experienced functional decline. In this study, at randomization, subjects will thus be enrolled in the trial based upon CAG repeat length and Baseline measures of the Symbol Digit Modality Test (SDMT), Total Motor Score (TMS), Independence Scale (IS) and Total Functional Capacity (TFC). These factors will be used to identify and enroll subjects with active disease who have not yet experienced functional decline, which may indicate a disease progression amenable to intervention. The Huntington's disease prognostic index ($PI_{HD}$) or its normed version ($PIN_{HD}$) score can be used to predict likelihood of HD progression. The PIN score will be calculated at Baseline to identify subjects eligible for participation in the study.

Based on the kinetics of Compound 1-mediated HTT lowering in humans, the maximal extent of tHTT protein lowering in HD patients is expected to be achieved between 4 and 6 weeks. The 12-week dosing regimen may further demonstrate that a steady-state decrease in tHTT is maintained over time with continued Compound 1 treatment in the Phase 2 Study, followed by a one year, open label extension. In addition to the primary endpoints of tHTT protein change from Baseline and safety, the Phase 2 study includes exploratory clinical outcome endpoints to assess the effect of Compound 1 on subjects' cognition and motor function as measured by the Unified Huntington's Disease Rating Scale (UHDRS). The UHDRS has been extensively studied and developed to assess disease progression in multiple domains. Cognitive impairment, motor function loss, and accelerated brain volume loss in the caudate and putamen are key features of this disorder and have a notable impact on quality of life. The assessment of more sensitive and early motor changes via wearable devices will also be included in the Phase 2 study as an exploratory endpoint. Studying these endpoints over 12 weeks will provide insight into the rate of change in earlier stages of disease and identify key measurements which may be early indicators of HD progression.

## Risk/Benefit Assessment

As described, HD is a relentlessly progressive, neurodegenerative disorder. Early in the course of the disease, patients exhibit subtle symptoms; as the disease progresses, involuntary writhing movements become more pronounced, voluntary motor capabilities decline, and speech and swallowing are increasingly impaired, while aggressive and disinhibited behavior become more frequent. Late-stage disease is marked by severe inability to walk, speak, swallow, or care for oneself, culminating in the need for full-time care and ultimately death, typically 15 to 18 years after the onset of symptoms (see, Caron, N, Wright, G and Hayden, M; (2020a), Huntington Disease; Seattle, WA; University of Washington).

There are currently no disease modifying interventions approved for use in HD and, without intervention, the patient population to be included in this trial will face continued disease progression, loss of function, and inevitably, death. The inexorable disease progression and inevitable mortality of the disease indicate that HD represents a high unmet medical need. Reduction of mHTT has been confirmed as an important therapeutic target.

As described above, in the Phase 1 study, multiple doses of Compound 1 were associated with marked reductions in HTT mRNA and protein. Pharmacokinetic-pharmacodynamic (PKPD) modeling based on interim data from the Phase 1 study determined that exposures at the 10 mg and 20 mg QD doses were associated with decreases in full-length *HTT* mRNA levels that precisely bookend the established mean 30% to 50% target range for HTT protein reduction. The 10 mg and 20 mg QD doses are thus anticipated to be associated with therapeutic benefit and the eventual slowing of disease progression in this Phase 2 study.

The Phase 1 study results provided evidence of Compound 1 safety and tolerability at single doses ranging from 5 mg to 135 mg and multiple doses of 15 mg and 30 mg for durations of up to 21 days. In this study, Compound 1 was safe and generally well tolerated. In both the single ascending dose (SAD) and multiple ascending dose (MAD) portions of the Phase 1 study, the overall incidence of AEs was comparable between subjects who received placebo and those who received Compound 1. There were no events considered to be dose-limiting toxicities, and all Adverse Events (AEs) were resolved at the time of the interim analysis cut-off date. There were also no clinically significant laboratory abnormalities or electrocardiogram (ECG) findings at any dose in either portion of the study.

The Phase 1 study will have a Data and Safety Monitoring Board (DSMB) that will closely monitor the safety of subjects. Based on the preclinical and clinical data to date, Compound 1 has a favorable risk/benefit profile in subjects with HD.

## Primary Study Objectives:

Evaluate the safety and pharmacodynamic effects of 2 treatment regimens of Compound 1 and placebo in subjects with Huntington's disease (HD) as assessed by: (i) Occurrence of treatment-emergent adverse events (TEAEs) and abnormalities in laboratory values, electrocardiogram (ECG), vital signs, slit lamp eye examination, and physical examination; and, (ii) Reduction in blood total huntingtin protein (HTT) levels. This aspect is intended to demonstrate the safety, tolerability and pharmacology of Compound 1 and reduction of HTT mRNA and HTT protein in HD patients.

## Secondary Study Objectives:

(i) Determine the effect of Compound 1 on *HTT* mRNA in blood and mHTT protein in cerebrospinal fluid (CSF); and, (ii) Reduction in blood mutant huntingtin protein (mHTT) levels. This aspect is intended to demonstrate the effect of Compound 1 on blood based, CSF-based and radiographic biomarkers of Huntington's disease.

## Exploratory Study Objectives:

(i) Assess the effect of Compound 1 on change in whole brain, caudate, and putamen volume via volumetric magnetic resonance imaging (vMRI); (ii) Assess the effect of change in ventricular volume via vMRI; (iii) Assess the effect of Compound 1 on plasma and CSF neurofilament light chain (NfL) protein concentrations; (iv) Assess change after 12 weeks of treatment in relevant scales, which will include an Assessment using the Unified Huntington's Disease Rating Scale (UHDRS) and each of its subcomponents, including (a) Symbol Digit Modalities Test (SDMT), (b) Total Motor Score (TMS), (c) Independence Scale, (d) Total Functional Capacity (TFC); (e) Gait and motor assessment via a wearable accelerometer; (f) Clinical Global Impression of Change (CGI-C); and, (g) Huntington's Disease Quality of Life questionnaire (HDQoL).

**Pharmacokinetic Objective:**

Evaluate the concentration of Compound 1 in subjects with HD.

**Clinical Endpoints:**

**Primary Safety Endpoints:**

Evaluate the safety profile as characterized by TEAEs, laboratory abnormalities, ECG, vital signs, slit lamp eye examination, and physical examination.

**Primary Efficacy Endpoint:**

Change from Baseline in blood total HTT protein at Visit 5.

**Biomarker Endpoints:**

(i) Percent reduction in HTT protein in CSF; (ii) Changes in neurofilament light chain (NfL) in plasma and CSF; and (iii) Change in caudate, putamenal, ventricular volume on volumetric MRI imaging.

**Secondary Endpoints:**

(i) Change from Baseline in blood *HTT* mRNA at Visits 3, 4, and 5; (ii) Change from Baseline in CSF mHTT at Visit 5; and, (iii) Change from Baseline in blood mHTT protein at Visit 5.

**Exploratory Endpoints:**

(i) Change from Baseline in whole brain, caudate, putamen, and ventricular volume (as assessed by vMRI); (ii) Change from Baseline in plasma and CSF NfL protein concentrations; (iii) Change from Baseline in UHDRS scores for each subscale, including the SDMT, TMS, Independence Scale, and TFC; (iv) Change from Baseline in total UHDRS; (v) Change from Baseline in wearable accelerometer assessment of gait and motor function; (vi) Assessment of change via the CGI-C; and, (vii) Change from Baseline in the HDQoL questionnaire.

**Pharmacokinetic Endpoint**

(i) Plasma trough concentration ($C_{trough}$) and accumulation ratio of plasma of Compound 1 on Visits 3, 4, and 5; and, (i) Accumulation ratio of Compound 1 in CSF on Visit 5.

**Biomarker Endpoints:**

(i) Percent reduction in HTT protein in CSF; (ii) Changes in neurofilament light chain (NfL) in plasma and CSF; and (iii) Change in caudate, putamenal, ventricular volume on volumetric MRI imaging.

**Study Design/Methodology:**

The Phase 2 Study is a randomized, placebo-controlled, parallel arm, dose-finding study to evaluate the safety and efficacy of 10 and 20 mg of Compound 1 and to determine the HTT protein lowering effect of these doses after 12 weeks of treatment in subjects with HD.

Individuals who sign an informed consent will enter screening to determine eligibility for the study. At Screening, potential subjects will have their gene mutation status confirmed by the Investigator (either via historical gene sequencing or through an in-study gene sequencing assessment) and undergo additional evaluation to confirm they meet the enrollment criteria. Subjects who satisfy all enrollment criteria at Screening will undergo baseline evaluations and be randomized to either 10 or 20 mg of study drug or placebo in a 1:1:1 randomization for a total of 12 weeks on treatment (plus or minus visit windows). Once assigned treatment, subjects will take their assigned dose of study medication, once a day, in the morning, at least 2 hours before their first meal of the day. Subjects will be asked to return to the clinic every 28 days after randomization (approximately Days 29, 57 and Day 85) or receive home care services in lieu of in-person visits to undergo study assessments. On Day 85, subjects will take their final dose of study medication and complete the end of study assessments. There will be a follow-up safety visit on Day 113 via telephone/telehealth to collect AEs.

**Sample Size Justification:**

The sample size calculation is based on mean change from Baseline in blood total HTT protein at Visit 5 (primary endpoint). Using effect size of 0.85 (i.e., the magnitude of treatment difference is 85% of one standard deviation), achievement of 90% power at 2-sided alpha level 0.05 would require 31 subjects. Assuming a 10% dropout rate, approximately 35 subjects will be randomized to each dose.

**Planned Number of Patients:**

Approximately 200 adult male and female subjects will be enrolled.

**Inclusion Criteria:**

Individuals eligible to participate in this study include those who meet all of the following inclusion criteria: (i) Ambulatory male or female patient aged 25 years and older, inclusive; (ii) Subject (or legally authorized representative) is willing and able to provide informed consent and comply with all protocol requirements; (iii) Genetically confirmed HD diagnosis with a cytosine-adenine-guanine (CAG) repeat length from 42 to 50, inclusive; (iv) A UHDRS-Independence Scale score of 100; (v) A TFC score of 13; (vi) A normed prognostic index for HD score between 0.18 to 4.93, inclusive; (vii) Women of childbearing potential (WOCBP): must agree to use highly effective methods of contraception during dosing and for 6 months after stopping the study medication.
WOCBP are defined as women who are fertile, following menarche and until becoming postmenopausal unless permanently sterile. Permanent sterilization methods include hysterectomy, bilateral salpingectomy, and bilateral oophorectomy. A postmenopausal state is defined as no menses for 12 months without an alternative medical cause. A high follicle stimulating hormone (FSH) level in the postmenopausal range may be used to confirm a postmenopausal state in women not using hormonal contraception or hormonal replacement therapy. However, in the absence of 12 months of amenorrhea, a single FSH measurement is insufficient. Highly effective contraception methods are defined as those that can achieve a failure rate of less than 1% per year when used consistently and correctly and include those selected from (a) combined (estrogen and progestogen containing) hormonal contraception associated with inhibition of ovulation, including contraception that is administered orally (WOCBP using oral contraception should have been stable on the same pill for a minimum of 3 months prior to Screening), intravaginally, or transdermally; (b) progestogen-only hormonal contraception associated with inhibition of ovulation, including contraception that is administered orally (WOCBP using oral contraception should have been stable on the same pill for a minimum of 3 months prior to Screening), via injectable, implantable, intrauterine device or intrauterine hormone-releasing system; or, (c) contraception associated with bilateral tubal occlusion, vasectomized partner or sexual abstinence.
(viii) Sexually active and fertile males must use a condom during intercourse while taking study drug and for 6 months after stopping study drug, and should neither father a child nor donate sperm in this period. A condom is required to be used also by vasectomized men in order to prevent potential delivery of the drug via seminal fluid.

**Main Criteria for Exclusion:**

Individuals are not eligible to participate in this study if they have met or meet any of the following exclusion criteria: (i) Inability or unwillingness to swallow oral tablets; (ii) Receipt of an experimental agent within 90 days or 5 half-lives prior to Screening or anytime over the duration of this study, including RNA- or DNA-targeted HD specific investigational agents, such as antisense oligonucleotides, cell transplantation, or any other experimental brain surgery; (iii) Any history of gene therapy exposure for the treatment of HD; (iv) Participation in an investigational trial or investigational paradigm (such as exercise/physical activity, cognitive therapy, brain stimulation, etc.) within 90 days prior to Screening or anytime over the duration of this study; (v) Presence of an implanted deep brain stimulation device; (vi) Family history of early onset cataracts or presence of cataracts at Baseline using a cataract grading system (Lens Opacities Classification System III) exam; (vii) Brain and spinal pathology that may interfere with CSF homeostasis and circulation, increased intracranial pressure (including presence of a shunt for the drainage of CSF or an implanted CNS catheter), malformations, and/or tumors; (viii) Hospitalization for any major medical or surgical procedure involving general anesthesia within 12 weeks of Screening or planned during the study; (ix) At significant risk of suicide as measured by the Columbia Suicide Severity Rating Scale (C-SSRS) with a moderate risk rating or higher score; (x) Risk of a major depressive episode, psychosis, confusional state, or violent behavior as assessed by the Investigator; (xi) Any medical history of brain or spinal disease that would interfere with the lumbar puncture process or safety assessments; (xii) History of malignancy of any organ system (other than localized basal cell carcinoma of the skin or in situ cervical cancer), treated or untreated, within the past 5 years, regardless of whether there is evidence of local recurrence or metastases; (xiii) Any medical history or condition that would interfere with the ability to complete the protocol-specified assessments (eg, implanted shunt, conditions precluding MRI scans); (xiv) Antidepressant or benzodiazepine use, unless receiving a stable dose for at least 6 weeks prior to Screening and with a dose regimen that is not anticipated to change during the study; (xvi) Lifetime history of drug or alcohol use in the high-risk category of risk drinking levels according to the World Health Organization for a duration of 1 month or longer as assessed by the Investigator; (xvii) Clinically significant medical condition, which in the opinion of the Investigator could adversely affect the safety of the subject or impair the assessment of study results; (xviii) Current significant renal impairment defined as estimated glomerular filtration rate <60 mL/min at Screening; (xvix) Current hepatic impairment resulting in elevated liver function test (aspartate transaminase, alanine transaminase, alanine phosphatase) at 3 times the upper limit of normal at Screening; (xx) Pregnancy, planning on becoming pregnant during the course of the trial, or currently breastfeeding; (xxi) Use of medications that are moderate or strong inhibitors of CYP3A4 within 1 week of Screening or medications that are moderate or strong inducers of CYP3A4 within 2 weeks of Screening or planned use of moderate or strong CYP3A4 inhibitor or inducer medications during the study period.

**Investigational and Reference Product, Dosage and Mode of Administration:**

Compound 1 tablets will be administered orally QD. The two investigation product dosing arms will be 10 mg for 12 weeks and 20 mg for 12 weeks.

Compound 1 active investigational product and matching placebo reference product tablets will be administered orally QD. Compound 1 investigational drug product is a film-coated tablet dosage form for oral administration. The white to off-white round coated tablets will be provided in 2 dosage strengths of 10 mg and 20 mg tablets which each contain Compound 1 drug substance and excipients selected from microcrystalline cellulose, lactose monohydrate, povidone K30, croscarmellose sodium, poloxamer 407, and magnesium stearate. The 10 mg and 20 mg tablets will be provided in 2 different sizes. The placebo tablet contains the same compendial excipients and is manufactured in the same tablet sizes with the same appearance to match the 10 mg and 20 mg Compound 1 tablets.

Evidence for the safety of the selected doses is provided by the ongoing Phase 1 study and the results of the comprehensive preclinical toxicology program to date. In the Phase 1 study, single doses ranging from 5 mg to 135 mg and multiple doses for 14 days of 15 mg and 30 mg have been safe and generally well tolerated.

A target 30% to 50% decrease in mHTT is the range associated with decreased pathology and anticipated therapeutic benefit in patients. In the Phase 1 study, Compound 1-mediated HTT pre-mRNA splicing was dose-dependent across all cohorts in both the SAD and MAD portions of the study. Mean decreases in full-length HTT mRNA levels of 40% and 60% were observed after 14 days of treatment with Compound 1 at 15 mg and 30 mg, respectively. On the basis of these clinical data, a PK-PD compartment model was used to simulate percentage of mRNA decreases (and thus the anticipated magnitude of HTT protein lowering) at additional potential clinical doses.

At the selected doses of 10 mg QD and 20 mg QD, the predicted percent full-length *HTT* mRNA decreases are within the target range of 30 to 50% reduction from baseline. Preclinical data in a bacterial artificial chromosome transgenic mouse model of HD, mice showed a strong correlation between levels of *HTT* pre-mRNA splicing and the degree of protein lowering following Compound 1 administration. Therefore, the observed preclinical *HTT* mRNA changes are anticipated to result in similar decreases in HTT protein levels in HD patients. Thus, based upon the totality of the clinical and preclinical safety data to date, and the anticipated reduction in HTT mRNA and protein derived from clinical data and pharmacokinetic-pharmacodynamic modeling, the doses of 10 mg and 20 mg are expected to be safe, well tolerated, and beneficial to subjects with HD.

**Reference Product, Dosage and Mode of Administration:**

Matching placebo tablets will be administered orally QD.

**Safety Criteria:**

Safety assessments will include observed TEAEs, clinical labs, vital signs, ECG, C-SSRS, slit lamp eye examination, and physical examination.

**Efficacy Criteria**:

Assessment of efficacy will include analysis of: (i) blood HTT protein and CSF NfL, (ii) UHDRS, (iii) CGI-C, (iv) wearable accelerometer for motor function, and (v) neuroimaging (vMRI).

**Enrichment Criteria**

Enrichment is defined as the prospective use of any patient characteristic to select a study population in which detection of a drug effect (if one is in fact present) is more likely than it would be in an unselected population. Due to the highly variable population of patients with HD, the enrichment strategy for this Phase 2 study is intended to select for subjects who have preserved capacity for activities of daily living, work, finances, and self-care, but have reduced performance on motor and cognitive tests and are predicted to experience functional impact on activities of daily living within 3 years. The TMS and SDMT from the UHDRS will be assessed at Screening (along with CAG repeat length and age) and used to identify this population via a validated HD prognostic index for pre-manifest HD patients.

The Huntington's disease prognostic index ($PI_{HD}$) or its normed version ($PIN_{HD}$) can be used to predict likelihood of HD progression, with higher scores indicating greater risk of functional decline. Natural history survival curves generated using the $PI_{HD}$ show the disease trajectory in patients with a particular $PI_{HD}$ score. The $PIN_{HD}$ score allows researchers to predict disease progression in a studied population with a high degree of certainty. Historically, disease progression was commonly indexed by the CAG-Age Product (CAP), which is a type of burden score of age and CAG expansion that has several variants. When CAP is supplemented with the TMS and SDMT from the UHDRS, predictive likelihood of HD progression increases. Using these enrichment criteria, a group of subjects with HD and no functional decline (measured via the TFC and IS) can be identified and changes in blood HTT levels after treatment can be measured. This group is likely to experience decline without HTT lowering treatment as it has been found that earlier stages of HD are marked by increases in mHTT levels in CSF compared to controls.

At Baseline in this study, subjects' cognitive and motor function will be assessed by SDMT and TMS scores, respectively. Enrolled subjects will present with no functional decline as assessed by the TFC and IS. Subjects will be included in the study based on the calculation of $PIN_{HD}$ scores as calculated by the IRT prior to randomization. Subjects with baseline $PIN_{HD}$ scores between 0.18 to 4.93 inclusive will be eligible for enrollment in the trial. The following formula will be utilized to calculate the $PIN_{HD}$ score:

$$PI_{HD} = 51 \times (TMS) + (-34) \times SDMT + 7 \times (age) \times (CAG - 34).$$

The $PI_{HD}$ score is converted to a normalized score using the following conversion:

$$PIN_{HD} = (PI_{HD} - 883)/1044$$

The ENROLL HD database (periodic data update 5) was utilized to identify the 0.18 to 4.93 range of $PIN_{HD}$ scores for inclusion in the study.

**Pharmacokinetics:**

Pharmacokinetic assessment will include plasma $C_{trough}$ (at Visits 3, 4, and 5). Accumulation ratio will be calculated and reported in plasma (Visits 3, 4, and 5) and CSF (Visit 5).

**Statistical Methods:**

A repeated measure analysis model (repeat on visit) will be used to compare each dose with placebo for blood total HTT protein. The model will include dose, visit, dose by visit interaction and baseline. Nominal p-values and 95% confidence interval for each pairwise comparison at Visit 5 (active versus placebo) will be provided. The model will include $PIN_{HD}$ as a stratification factor. The same analysis used for blood HTT protein will be used for blood *HTT* mRNA. Dose-response relationships will be explored. Demographic and baseline characteristics, disposition, safety, and efficacy endpoints will be summarized descriptively by dose group. Statistical models will be applied to understand the relationship between UHDRS and its components to blood and CSF assessments.

**Phase 2 Study Results**

The primary objective of the 12 week Phase 2a, randomized, placebo-controlled, dose-finding study is to evaluate the safety and pharmacodynamic effects of two treatment regimens of Compound 1 and placebo in subjects with Huntington's Disease. The primary objective assesses the occurrence of treatment-emergent adverse events (TEAEs); abnormalities in laboratory values, electrocardiogram (ECG), vital signs, slit lamp eye examination, and physical examination; and, reduction in blood total huntingtin protein (HTT) levels.

The secondary objectives of the study determine the effect of Compound 1 on *HTT* mRNA in blood and mHTT protein in cerebrospinal fluid (CSF); and, reduction in blood mutant huntingtin protein (mHTT) levels.

The exploratory objectives of the study assess the effect of Compound 1 on change in whole brain, caudate, and putamen volume via volumetric magnetic resonance imaging (vMRI); assess the effect of change in ventricular volume via vMRI; assess the effect of Compound 1 on plasma and CSF neurofilament light chain (NfL) protein concentrations; assess change after 12 weeks of treatment in relevant scales, which will include an assessment using the Unified Huntington's Disease Rating Scale (UHDRS) and each of its subcomponents. The UHDRS subcomponents are used to assess qualitative efficacy including, (a) Symbol Digit Modalities Test (SDMT), (b) Total Motor Score (TMS), (c) Independence Scale; (d) Total Functional Capacity (TFC); (e) Gait and motor assessment via a wearable accelerometer; (f) Clinical Global Impression of Change (CGI-C); and, (g) Huntington's Disease Quality of Life questionnaire (HDQoL).

The pharmacokinetic objectives of the study evaluate the concentration of Compound 1 in subjects with HD.

## Claims

1. A tablet comprising, as an active ingredient, 2-[3-(2,2,6,6-tetramethylpiperidin-4-yl)-3H-[1,2,3]triazolo[4,5-c]pyridazin-6-yl]-5-(2H-1,2,3-triazol-2-yl)phenol (hereinafter Compound 1), or a pharmaceutically acceptable salt thereof, wherein Compound 1 is present in an amount from about 5% to about 30% by weight of the total weight of the tablet, an intragranular excipient, and an extragranular excipient; wherein the intragranular excipient comprises microcrystalline cellulose and a diluent; wherein the ratio of microcrystalline cellulose to diluent is about 1:1 to about 1:4 and the microcrystalline cellulose is present in an amount of about 15% to about 25% by weight of the total weight of the tablet; wherein the disintegrant is present in an amount of about 1% to about 3% of the total weight of the tablet; wherein povidone is present in an amount of 1% to about 5% by weight of the total weight of the tablet; and, wherein the extragranular excipient comprises an additional amount of the diluent and an additional amount of the disintegrant.

2. The tablet of claim 1, wherein Compound 1 is present in an amount of about 5% to about 25% of the total weight of the tablet; or,

   wherein Compound 1 is about 10% of the total weight of the tablet; or,
   wherein the ratio of microcrystalline cellulose to diluent in the intragranular excipient is about 1:2.

3. The tablet of claim 1, wherein the amount of Compound 1 in the tablet is about 1 mg to 200 mg, preferably the amount of Compound 1 in the tablet is 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, or 200 mg, more preferably the amount of Compound 1 is in the tablet is about 1 mg to about 100 mg, and most preferably the amount of Compound 1 in the tablet is 1 mg, 5 mg, 10 mg, 15 mg, 20 mg, 25 mg, 50 mg, or 100 mg.

4. The tablet of any of claims 1-3, wherein the diluent is lactose monohydrate.

5. The tablet of any of claims 1-4, wherein the extragranular diluent is present in an amount of about 15% to about 30% of the total weight of the tablet.

6. The tablet of any of claims 1-5, wherein the at least one of the extragranular excipient and the intragranular excipient further comprises a surfactant, and wherein the surfactant is preferably a poloxamer.

7. The tablet of any of claims 1-6, wherein the disintegrant is croscarmellose sodium.

8. The tablet of any of claims 1-7, wherein the extragranular excipient further comprises a lubricant, and wherein the lubricant is preferably magnesium stearate; or,
   wherein the extragranular excipient further comprises a glidant., and wherein the glidant is preferably colloidal silicon dioxide.

9. The tablet of any of claims 1-8, wherein the weight of the extragranular excipient is from about 15% to about 30% by weight of the total weight of the tablet.

10. The tablet of any of claims 1-9, wherein the intragranular excipients have been wet granulated.

11. The tablet of any of claims 1-10, wherein Compound 1 is present in an amount of 10% by weight of the tablet, the intragranular excipient comprises microcrystalline cellulose and lactose monohydrate in a ratio of about 1:2 and the microcrystalline cellulose is present in an amount of about 20% by weight of the tablet, the disintegrant in an amount of about 1% to about 3% by weight of the tablet, and the povidone in an amount of about 2% by weight of the tablet, wherein the extragranular excipient comprises the lactose monohydrate in an amount of about 10% to about 25% by weight of the tablet, the disintegrant in an amount of about 1% to about 5% by weight of the tablet, and poloxamer in an amount of about 0.5% to about 2% by weight of the tablet.

12. The tablet of any of claims 1-11, wherein the extragranular excipient further comprises colloidal silicon dioxide in an amount of about 0.25 % to about 1% by weight of the tablet.

13. The tablet of any of claims 1-12, wherein the extragranular excipient further comprises magnesium stearate in an amount of about 0.5% to about 2% by weight of the tablet.

14. A method of making the tablet of any of claims 1-13, comprising wet granulating the extragranular excipients, drying the resulting intragranular blend, mixing the extragranular excipient with the intragranular excipient, compressing the resulting mixture to form a tablet, and optionally coating the resulting tablet with a film.

15. A tablet according to any of claims 1-13 for use in a method of treating or ameliorating Huntington's Disease in a subject in need thereof, wherein the method comprises orally administering to the subject the tablet according to any one of claims 1-13, or a pharmaceutically acceptable salt thereof, and preferably wherein the tablet contains a therapeutically effective amount of Compound 1 in a range of from 1 mg to 200 mg, more preferably the tablet contains a therapeutically effective amount of Compound 1 in a range of from 1 mg to 100 mg, more preferably the tablet contains 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, or 200 mg of Compound 1, and most preferably the tablet contains 1 mg, 5 mg, 10 mg, 15 mg, 20 mg, 25 mg or 100 mg of Compound 1.

**Patentansprüche**

1. Tablette, umfassend als Wirkstoff 2-[3-(2,2,6,6-Tetramethylpiperidin-4-yl)-3H-[1,2,3]triazolo[4,5-c]pyridazin-6-yl]-5-(2H-1,2,3-triazol-2-yl)phenol (im Folgenden als Verbindung 1 bezeichnet) oder ein pharmazeutisch unbedenkliches Salz davon, wobei Verbindung 1 in einer Menge von etwa 5 Gew.-% bis 30 Gew.-% des Gesamtgewichts der Tablette vorhanden ist, einen intragranulären Hilfsstoff und einen extragranulären Hilfsstoff; wobei der intragranuläre Hilfsstoff mikrokristalline Cellulose und ein Verdünnungsmittel umfasst; wobei das Verhältnis von mikrokristalliner Cellulose zu Verdünnungsmittel etwa 1:1 bis etwa 1:4 beträgt und die mikrokristalline Cellulose in einer Menge von etwa 15 Gew.-% bis etwa 25 Gew.-% des Gesamtgewichts der Tablette vorhanden ist; wobei das Sprengmittel in einer Menge von etwa 1 % bis etwa 3 % des Gesamtgewichts der Tablette vorhanden ist; wobei Povidon in einer Menge von 1 Gew.-% bis etwa 5 Gew.-% des Gesamtgewichts der Tablette vorhanden ist; und wobei der extragranuläre Hilfsstoff eine zusätzliche Menge des Verdünnungsmittels und eine zusätzliche Menge des Sprengmittels umfasst.

2. Tablette nach Anspruch 1, wobei Verbindung 1 in einer Menge von etwa 5 % bis etwa 25 % des Gesamtgewichts der Tablette vorhanden ist; oder

   wobei Verbindung 1 etwa 10 % des Gesamtgewichts der Tablette ausmacht; oder
   wobei das Verhältnis von mikrokristalliner Cellulose zu Verdünnungsmittel in dem extragranulären Hilfsstoff etwa 1:2 beträgt.

3. Tablette nach Anspruch 1, wobei die Menge von Verbindung 1 in der Tablette etwa 1 mg bis 200 mg beträgt, bevorzugt wobei die Menge von Verbindung 1 in der Tablette 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg oder 200 mg beträgt, weiter bevorzugt wobei die Menge von Verbindung 1 in der Tablette etwa 1 mg bis etwa 100 mg beträgt, und ganz besonders bevorzugt wobei die Menge von Verbindung 1 in der Tablette 1 mg, 5 mg, 10 mg, 15 mg, 20 mg, 25 mg, 50 mg oder 100 mg beträgt.

**4.** Tablette nach einem der Ansprüche 1-3, wobei es sich bei dem Verdünnungsmittel um Lactosemonohydrat handelt.

**5.** Tablette nach einem der Ansprüche 1-4, wobei das extragranuläre Verdünnungsmittel in einer Menge von etwa 15 % bis etwa 30 % des Gesamtgewichts der Tablette vorhanden ist.

**6.** Tablette nach einem der Ansprüche 1-5, wobei der extragranuläre Hilfsstoff oder der intragranuläre Hilfsstoff ferner ein Tensid umfasst und wobei es sich bei dem Tensid bevorzugt um ein Poloxamer handelt.

**7.** Tablette nach einem der Ansprüche 1-6, wobei es sich bei dem Sprengmittel um Croscarmellose-Natrium handelt.

**8.** Tablette nach einem der Ansprüche 1-7, wobei der extragranuläre Hilfsstoff ferner ein Schmiermittel umfasst und wobei es sich bei dem Schmiermittel bevorzugt um Magnesiumstearat handelt; oder wobei der extragranuläre Hilfsstoff ferner ein Gleitmittel umfasst und wobei es sich bei dem Gleitmittel bevorzugt um kolloidales Siliciumdioxid handelt.

**9.** Tablette nach einem der Ansprüche 1-8, wobei das Gewicht des extragranulären Hilfsstoffs etwa 15 Gew.-% bis etwa 30 Gew.-% des Gesamtgewichts der Tablette ausmacht.

**10.** Tablette nach einem der Ansprüche 1-9, wobei die intragranulären Hilfsstoffe nassgranuliert wurden.

**11.** Tablette nach einem der Ansprüche 1-10, wobei Verbindung 1 in einer Menge von 10 Gew.-% der Tablette vorhanden ist, der intragranuläre Hilfsstoff mikrokristalline Cellulose und Lactosemonohydrat in einem Verhältnis von etwa 1:2 umfasst und die mikrokristalline Cellulose in einer Menge von etwa 20 Gew.-% der Tablette vorhanden ist, das Sprengmittel in einer Menge von etwa 1 Gew.-% bis etwa 3 Gew.-% der Tablette vorhanden ist und das Povidon in einer Menge von etwa 2 Gew.-% der Tablette vorhanden ist, wobei der extragranuläre Hilfsstoff das Lactosemonohydrat in einer Menge von etwa 10 Gew.-% bis etwa 25 Gew.-% der Tablette, das Sprengmittel in einer Menge von etwa 1 Gew.-% bis etwa 5 Gew.-% der Tablette und Poloxamer in einer Menge von etwa 0,5 Gew.-% bis etwa 2 Gew.-% der Tablette umfasst.

**12.** Tablette nach einem der Ansprüche 1-11, wobei der extragranuläre Hilfsstoff ferner kolloidales Siliciumdioxid in einer Menge von etwa 0,25 Gew.-% bis etwa 1 Gew.-% der Tablette umfasst.

**13.** Tablette nach einem der Ansprüche 1-12, wobei der extragranuläre Hilfsstoff ferner Magnesiumstearat in einer Menge von etwa 0,5 Gew.-% bis etwa 2 Gew.-% der Tablette umfasst.

**14.** Verfahren zum Herstellen der Tablette nach einem der Ansprüche 1-13, umfassend Nassgranulieren der extragranulären Hilfsstoffe, Trocknen des resultierenden intragranulären Gemischs, Vereinigen des extragranulären Hilfsstoffs mit dem intragranulären Hilfsstoff, Verpressen der resultierenden Mischung zur Bildung einer Tablette und gegebenenfalls Beschichten der resultierenden Tablette mit einem Film.

**15.** Tablette gemäß einem der Ansprüche 1-13 zur Verwendung bei einem Verfahren zum Behandeln oder Lindern von Morbus Huntington in einem Individuum, das diesbezüglich Bedarf hat, wobei das Verfahren orales Verabreichen der Tablette gemäß einem der Ansprüche 1-13 oder eines pharmazeutisch unbedenklichen Salzes davon an das Individuum umfasst und bevorzugt wobei die Tablette eine therapeutische wirksame Menge von Verbindung 1 in einem Bereich von 1 mg bis 200 mg enthält, weiter bevorzugt die Tablette eine therapeutisch wirksame Menge von Verbindung 1 in einem Bereich von 1 mg bis 100 mg enthält, weiter bevorzugt die Tablette 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg oder 200 mg Verbindung 1 enthält und ganz besonders bevorzugt die Tablette 1 mg, 5 mg, 10 mg, 15 mg, 20 mg, 25 mg oder 100 mg Verbindung 1 enthält.

**Revendications**

**1.** Comprimé comprenant, comme ingrédient actif, du 2-[3-(2,2,6,6-tétraméthylpipéridin-4-yl)-3H-[1,2,3]triazolo[4,5-c] pyridazin-6-yl]-5-(2H-1,2,3-triazol-2-yl)phénol (ci-après Composé 1), ou un sel pharmaceutiquement acceptable correspondant, dans lequel le Composé 1 est présent en une quantité d'environ 5 % à environ 30 % en poids du poids total du comprimé, un excipient intragranulaire et un excipient extragranulaire ; l'excipient intragranulaire comprenant une cellulose microcristalline et un diluant ; dans lequel le rapport de la cellulose microcristalline sur le diluant est d'environ 1:1 à environ 1:4 et la cellulose microcristalline est présente en une quantité d'environ 15 % à environ 25 % en poids du poids total du comprimé ; dans lequel le désintégrant est présent en une quantité d'environ 1 % à environ 3 % du poids total du comprimé ; dans lequel une povidone est présente en une quantité de 1 % à environ 5 % en poids du poids total du comprimé ; et, dans lequel l'excipient extragranulaire comprend une quantité supplémentaire du diluant et une quantité supplémentaire du désintégrant.

2. Comprimé selon la revendication 1, dans lequel le Composé 1 est présent en une quantité d'environ 5 % à environ 25 % du poids total du comprimé ; ou,

dans lequel le Composé 1 représente environ 10 % du poids total du comprimé ; ou,
dans lequel le rapport de cellulose microcristalline sur diluant dans l'excipient intragranulaire est d'environ 1:2.

3. Comprimé selon la revendication 1, dans lequel la quantité de Composé 1 dans le comprimé est d'environ 1 mg à 200 mg, de préférence la quantité de Composé 1 dans le comprimé est de 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg ou 200 mg, plus préférablement la quantité de Composé 1 dans le comprimé est d'environ 1 mg à environ 100 mg, et le plus préférablement la quantité de Composé 1 dans le comprimé est de 1 mg, 5 mg, 10 mg, 15 mg, 20 mg, 25 mg, 50 mg ou 100 mg.

4. Comprimé selon l'une quelconque des revendications 1 à 3, dans lequel le diluant est du monohydrate de lactose.

5. Comprimé selon l'une quelconque des revendications 1 à 4, dans lequel le diluant extragranulaire est présent en une quantité d'environ 15 % à environ 30 % du poids total du comprimé.

6. Comprimé selon l'une quelconque des revendications 1 à 5, dans lequel l'au moins un parmi l'excipient extragranulaire et l'excipient intragranulaire comprend en outre un tensioactif, et dans lequel le tensioactif est de préférence un poloxamère.

7. Comprimé selon l'une quelconque des revendications 1 à 6, dans lequel le désintégrant est une croscarmellose sodique.

8. Comprimé selon l'une quelconque des revendications 1 à 7, dans lequel l'excipient extragranulaire comprend en outre un lubrifiant, et dans lequel le lubrifiant est de préférence le stéarate de magnésium ; ou, dans lequel l'excipient extragranulaire comprend en outre un agent glissant, et dans lequel l'agent glissant est de préférence du dioxyde de silicium colloïdal.

9. Comprimé selon l'une quelconque des revendications 1 à 8, dans lequel le poids de l'excipient extragranulaire est d'environ 15 % à environ 30 % en poids du poids total du comprimé.

10. Comprimé selon l'une quelconque des revendications 1 à 9, dans lequel les excipients intragranulaires ont été granulés par voie humide.

11. Comprimé selon l'une quelconque des revendications 1 à 10, dans lequel le Composé 1 est présent en une quantité de 10 % en poids du comprimé, l'excipient intragranulaire comprend une cellulose microcristalline et du monohydrate de lactose en un rapport d'environ 1:2 et la cellulose microcristalline est présente en une quantité d'environ 20 % en poids du comprimé, le désintégrant en une quantité d'environ 1 % à environ 3 % en poids du comprimé, et la povidone en une quantité d'environ 2 % en poids du comprimé, dans lequel l'excipient extragranulaire comprend le monohydrate de lactose en une quantité d'environ 10 % à environ 25 % en poids du comprimé, le désintégrant en une quantité d'environ 1 % à environ 5 % en poids du comprimé, et le poloxamère en une quantité d'environ 0,5 % à environ 2 % en poids du comprimé.

12. Comprimé selon l'une quelconque des revendications 1 à 11, dans lequel l'excipient extragranulaire comprend en outre du dioxyde de silicium colloïdal en une quantité d'environ 0,25 % à environ 1 % en poids du comprimé.

13. Comprimé selon l'une quelconque des revendications 1 à 12, dans lequel l'excipient extragranulaire comprend en outre du stéarate de magnésium en une quantité d'environ 0,5 % à environ 2 % en poids du comprimé.

**14.** Procédé de fabrication du comprimé selon l'une quelconque des revendications 1 à 13, comprenant la granulation humide des excipients extragranulaires, le séchage du mélange intragranulaire résultant, le mélange de l'excipient extragranulaire avec l'excipient intragranulaire, la compression du mélange résultant pour former un comprimé, et éventuellement l'enrobage du comprimé résultant avec un film.

**15.** Comprimé selon l'une quelconque des revendications 1 à 13 pour une utilisation dans un procédé de traitement ou d'amélioration de la maladie de Huntington chez un sujet qui en a besoin, dans lequel le procédé comprend l'administration orale au sujet du comprimé selon l'une quelconque des revendications 1 à 13, ou d'un sel pharmaceutiquement acceptable correspondant, et de préférence, dans lequel le comprimé contient une quantité thérapeutiquement efficace du Composé 1 dans une plage de 1 mg à 200 mg, plus préférablement le comprimé contient une quantité thérapeutiquement efficace du Composé 1 dans une plage de 1 mg à 100 mg, plus préférablement le comprimé contient 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg ou 200 mg du Composé 1, et le plus préférablement le comprimé contient 1 mg, 5 mg, 10 mg, 15 mg, 20 mg, 25 mg ou 100 mg du Composé 1.

**FIG. 1**

FIG. 2

**FIG. 3**

**FIG. 4**

FIG. 5

FIG. 6

FIG. 7

FIG. 8

FIG. 9A

FIG. 9B

**1 Initial steady state:**
100% HTT mRNA & protein

**2 Add splicing drug** (compound I)
target dose where 50% of new mRNA will contain STOP-exon

**3 50% HTT protein produced:**
HTT protein T½ is longer than T½ HTT mRNA

50% HTT mRNA
+
50% STOP-exon (degraded)

**4 New steady state reached:**
50% HTT mRNA & protein

concentration

time

**FIG. 10**

**HTT mRNA abundance**
**Steady state lowering should reach within 1 week**

FIG. 11A

HTT protein abundance
Steady state lowering should take ≥6 weeks

FIG. 11B

HTT mRNA abundance
Steady state lowering reached within 1 week

HTT protein abundance
Steady state lowering should take ≥6 weeks

FIG. 12A

FIG. 12B

Human plasma-CSF correlation

Monkey plasma-CSF correlation

**FIG. 13A**

**FIG. 13B**

FIG. 14

FIG. 15

EP 4 243 782 B1

# Huntingtin mRNA and Protein Levels in Blood (MAD2.3) QDX21

**FIG. 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63113826
- US 63245927
- US 63261467
- US 63261495
- US 63255745
- WO 2020005873 A

**Non-patent literature cited in the description**

- *European Journal of Neurology*, 2017, 24-34
- *Neurology*, 1979, vol. 29, 1-3
- *Neurology*, 1981, vol. 31, 1333-1335
- *Movement Disorders*, 1996, vol. 11, 136-142
- *Lancet Neural.*, 2013, vol. 12 (7), 637-649
- *Movement Disorders*, 2014, vol. 29 (10), 1281-1288
- *Movement Disorders*, 2016, vol. 31 (10), 1466-1478
- *Movement Disorders*, 2015, vol. 30 (14), 1954-1960
- *Neurology*, 2017, vol. 89, 2495-2502
- *Movement Disorders*, 2018, vol. 33 (5), 742-749
- **CARON, N** ; **WRIGHT, G** ; **HAYDEN, M**. Huntington Disease; Seattle, WA. University of Washington, 2020